(19) Europäisches Patentamt
European Patent Office
Office européen des brevets

(11) Veröffentlichungsnummer: **0 268 585**
**B1**

(12) # EUROPÄISCHE PATENTSCHRIFT

(45) Veröffentlichungstag der Patentschrift: **30.05.90**

(51) Int. Cl.$^5$: **C 07 D 473/04,** A 61 K 31/52, C 07 D 473/06, C 07 D 473/08

(21) Anmeldenummer: **86904158.2**

(22) Anmeldetag: **08.07.86**

(86) Internationale Anmeldenummer: **PCT/EP86/00401**

(87) Internationale Veröffentlichungsnummer: **WO 87/00523 29.01.87 Gazette 87/03**

(54) **TERTIÄRE HYDROXYALKYLXANTHINE, VERFAHREN ZU IHRER HERSTELLUNG, DIE SIE ENTHALTENDEN ARZNEIMITTEL UND IHRE VERWENDUNG.**

(30) Priorität: **19.07.85 DE 3525801**

(43) Veröffentlichungstag der Anmeldung: **01.06.88 Patentblatt 88/22**

(45) Bekanntmachung des Hinweises auf die Patenterteilung: **30.05.90 Patentblatt 90/22**

(84) Benannte Vertragsstaaten: **AT BE CH DE FR GB IT LI LU NL SE**

(56) Entgegenhaltungen:
**DE-A-1 245 969**
**DE-A-2 207 860**
**DE-A-2 432 702**
**FR-A-1 211 333**
**FR-A-2 236 501**
**US-A-2 517 410**

(73) Patentinhaber: **HOECHST AKTIENGESELLSCHAFT**
**Postfach 80 03 20**
**D-6230 Frankfurt am Main 80 (DE)**

(72) Erfinder: **GEBERT, Ulrich**
**Albert-Lortzing-Strasse 2**
**D-6233 Kelkheim (DE)**
Erfinder: **OKYAYUZ-BAKLOUTI, Ismahan**
**Ernst-von-Harnack-Strasse 18**
**D-6200 Wiesbaden (DE)**
Erfinder: **THORWART, Werner**
**Am Gänsborn 3b**
**D-6203 Hochheim am Main (DE)**

**EP 0 268 585 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die vorliegende Erfindung betrifft neue Xanthinderivate mit mindestens einer tertiären Hydroxyalkylgruppe in 1- oder 7-Stellung, Verfahren zu deren Herstellung und deren Verwendung als Wirkstoffe in Arzneimitteln, die sich insbesondere zur Behandlung von peripheren und cerebralen Durchblutungsstörungen eignen.

Es sind bereits durchblutungsfördernd wirkende 1-Oxoalkyl-3,7-dialkyl- und 7-Oxoalkyl-1,3-dialkylxanthine bzw. 1-Hydroxyalkyl-3,7-dialkyl- und 7-Hydroxyalkyl-1,3-dialkylxanthine mit sekundärer Alkoholfunktion bekannt. Innerhalb dieser Stoffgruppe hat das Vasotherapeutikum Pentoxifyllin, das 3,7-Dimethyl-1-(5-oxohexyl)-xanthin, beachtliche therapeutische Bedeutung für die medikamentöse Behandlung sowohl peripherer als auch cerebraler Durchblutungsstörungen erlangt. Als vasoaktives Medikament der neueren Generation (Schweiz. med. Wschr. *111* (1981), S. 637—640) besitzt es heute in vielen Ländern beispielsweise einen festen Platz unter den zur Therapie der peripheren arteriellen Verschlußkrankheit angewandten Arzneimitteln, während es in einigen Ländern mit großem Erfolg auch bei cerebralem Durchblutungsmangel eingesetzt wird.

Der klinisch gut etablierten Wirkung dieses Präparates steht jedoch der Nachteil gegenüber, daß sowohl der Wirkstoff per se als auch sein erster, ebenfalls pharmakologisch aktiver Metabolit, das 1-(5-Hydroxyhexyl)-3,7-dimethylxanthin, einer schnellen und vollständigen Biotransformation bei Tier und Mensch unterliegen, die fast ausschließlich über die enzymatische Oxydation der Oxo- bzw. Hydroxyhexyl-Seitenkette verläuft und mit einem ausgeprägten "Leber-first-pass"-Effekt verbunden ist. Dies bedeutet, daß vor allem bei oraler Applikation ein erheblicher Anteil der verabreichten Dosis nach Absorption aus dem Magen-Darm-Trakt und Transport über das Pfortadersystem zur Leber, dem wichtigsten Filterorgan für Fremdstoffe, bereits im Verlaufe der ersten Leberpassage von arzneimittelabbauenden Enzymen metabolisiert wird, so daß trotz vollständiger Absorption nur ein bestimmter Teil des Pharmakons in unveränderter Form den systemischen, großen Blutkreislauf erreicht. Der "First-pass"-Effekt, auch präsystemische Elimination genannt, führt somit zu einer Verminderung der systemischen Verfügbarkeit an unveränderter Wirksubstanz. Das eigentliche Handikap eines ausgeprägten "First-pass"-Effektes besteht jedoch weniger darin, daß die orale Dosis auf dem Weg in die systemische Zirkulation reduziert wird, sondern daß dieser Vorgang in der Regel eine große intra- und interindividuelle Variabilität aufweist (Schweiz. med. Wschr. 110 (1980) S. 354—362), die die Aufstellung verbindlicher Dosierungsschemata erschwert und damit den Therapieerfolg beeinträchtigen kann.

Dieser unbefriedigende Sachverhalt begründet den verständlichen Wunsch der Kliniker und die intensive Suche der pharmazeutischen Forschung nach neuen Xanthinverbindungen, die bei ähnlich guter oder womöglich noch stärkerer pharmakologischer Wirkung und gleicher hervorragender Verträglichkeit eine deutlich größere metabolische Stabilität besitzen, damit einen wesentlich geringeren oder gar vernachlässigbaren "First-pass"-Effekt aufweisen und folglich die Therapiesicherheit bezüglich der zuvor beschriebenen Dosierungsprobleme entscheidend verbessern. Derartige Präparate würden einen echten Fortschritt in der medikamentösen Therapie peripherer und cerebraler Durchblutungsstörungen darstellen, die in den industrialisierten Ländern zu den häufigsten Krankheits- und Todesursachen zählen.

Oberraschend wurde nun gefunden, daß die bislang nicht untersuchte Alkylverzweigung des sekundären Hydroxyalkylrestes an dem die Hydroxylgruppe tragenden Kohlenstoffatom unabhängig von der Stellung dieses Restes am Xanthingerüst in 1- und/oder 7-Position zu Verbindungen führt, deren Hydroxyalkyl-Seitenkette mit nunmehr tertiärer Alkoholstruktur gegenüber den mischfunktionellen mikrosomalen Oxydasen der Leber stabil ist und die gleichzeitig auch die anderen vorgenannten therapeutischen Anforderungen erfüllen.

Gegenstand der vorliegendern Erfindung sind somit tertiäre Hydroxyalkylxanthine der allgemeinen Formel I (s. Patentanspruch 1), in der mindestens einer der Reste

$R^1$ und $R^3$ eine verzweigte Hydroxyalkylgruppe der Formel

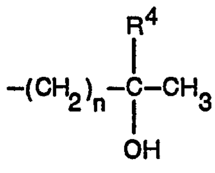

(Ia)

mit tertiärer Alkoholfunktion darstelt, wobei $R^4$ eine Alkylgruppe mit 1 bis 3 C-Atomen und n eine ganze Zahl von 2 bis 5 bedeuten, der gegebenenfalls vorhandene andere Rest

$R^1$ oder $R^3$ für ein Wasserstoffatom oder einen aliphatischen Kohlenwasserstoff-Rest $R^5$ mit bis zu 6 C-Atomen steht, dessen Kohlenstoffkette von bis zu 2 Sauerstoffatomen unterbrochen oder mit einer Oxogruppe oder bis zu zwei Hydroxygruppen substituiert sein kann, und

$R^2$ eine Alkylgruppe mit 1 bis 4 C-Atomen darstellt.

Bevorzugt sind dabei solche Verbindungen der Formel I, bei denen $R^2$ für eine Methyl- oder Äthylgruppe steht. Gleichermaßen bevorzugt sind jene Verbindungen der Formel I, in denen nur einer der beiden Reste $R^1$ oder $R^3$ die vorstehend definierte tertiäre Hydroxyalkylgruppe darstellt. Weiterhin bevorzugt sind solche Verbindungen, bei denen $R^4$ für eine Methylgruppe steht und n eine ganze Zahl von 3

bis 5 bedeutet, so daß der tertiäre Hydroxyalkylrest Ia entweder [(ω-1)-Hydroxy-(ω-1)-methyl]-pentyl, -hexyl oder -heptyl darstellt, insbesondere solche, bei denen $R^2$ Methyl oder Äthyl bedeutet. Weiterhin sind diejenigen Verbindungen der Formel I besonders hervorzuheben, in denen $R^1$ die tertiäre Hydroxyalkylgruppe darstellt und $R^3$ für Alkyl, Hydroxyalkyl oder Alkoxyalkyl mit jeweils 1 bis 4 C-Atomen steht, wie etwa das 7-Äthoxymethyl-1-(5-hydroxy-5-methylhexyl)-3-methyl-xanthin.

Repräsentative Reste für die Gruppe $R^5$ in der Position von $R^1$ oder $R^3$ sind beispielsweise Methyl, Äthyl, Propyl, Isopropyl, Butyl, Isobutyl, Pentyl und Hexyl, deren Hydroxy- und Oxo-Derivate, deren Hydroxy-bzw. Oxogruppe durch mindestens 2 C-Atome vom Stickstoff-getrennt ist, wie Hydroxyäthyl, 2- und 3-Hydroxypropyl, 2,3-Dihydroxypropyl, 2-, 3- und 4-Hydroxybutyl, 2-Hydroxy-2-methylpropyl, 3,4-Dihydroxybutyl, 4,5-bzw. 3,4-Dihydroxypentyl, 5,6- bzw. 4,5-Dihydroxyhexyl, 4-Hydroxypentyl, 5-Hydroxyhexyl, 2-Oxopropyl, 3-Oxobutyl, 4-Oxopentyl und 5-Oxohexyl, sowie die Alkoxyalkyl- und Alkoxyalkoxyalkylgruppen, wie etwa Methoxy-methyl, -äthyl und -propyl, Äthoxy-methyl, -äthyl und -propyl, Propoxymethyl und -äthyl, Methoxyäthoxymethyl und -äthyl und Äthoxyäthoxy-methyl und -Äthyl.

Gegenstand der Erfindung ist weiterhin ein Verfahren zur Herstellung der neuen tertiären Hydroxyalkylxanthine.

Eine Ausführungsform besteht beispielsweise darin, daß man

a) 3-Alkylxanthine der Formel II (s. Patentanspruch 6), in der $R^2$ Alkyl mit bis zu 4 C-Atomen darstellt, mit Alkylierungsmitteln der Formel III (s. Patentanspruch 6), in der X Halogen, vorzugsweise Chlor, Brom oder Jod, oder eine Sulfonsäureester- oder Phosphorsäureester-Gruppierung ist und $R^4$ und n die oben genannten Bedeutungen haben, zu erfindungsgemäßen Verbindungen der Formel I b (s. Patentanspruch 6) mit einer tertiären Hydroxyalkylgruppe in der Position von $R^3$ und Wasserstoff in der Position von $R^1$ umsetzt, und diese

$a_1$) mit demselben oder einem anderen Alkylierungsmittel der Formel III zu erfindungsgemäßen Verbindungen der Formel I c (s. Patentanspruch 6) mit zwei gleichen oder verschiedenen tertiären Hydroxyalkylgruppen in den Positionen von $R^1$ und $R^3$ alkyliert oder

$a_2$) durch Umsetzung mit einer Verbindung der Formel $R^5$-X (IV), in der X die bei Formel III und $R^5$ die oben angegebene Bedeutung haben, in erfindungsgemäße Verbindungen der Formel I d (s. Patentanspruch 6) umwandelt, wobei in allen Fällen zweckmäßig in Gegenwart basischer Mittel gearbeitet wird oder die Xanthine in Form ihrer Salze eingesetzt werden.

Eine andere Ausführungsform b) besteht darin, daß man 1,3-dialkylierte Xanthine der Formel V (s. Patentanspruch 6), zweckmäßig in Gegenwart basischer Mittel oder in Form ihrer Salze, durch einstufige Umsetzung mit einer Verbindung der Formel III in der 7-Stellung zu Verbindungen der Formel Id substituiert.

Eine weitere Ausführungsform c) besteht darin, daß man die 3-Alkylxanthine der Formel II, ebenfalls vorzugsweise in Gegenwart basischer Mittel oder in Form ihrer Salze, zuerst mit einer Verbindung der Formel $R^6$-X (IVa) unter Bildung von 3,7-disubstituierten Xanthinen der Formel VI (s. Patentanspruch 6), in der $R^6$ die für $R^5$ genannte Bedeutung hat oder Benzyl oder Diphenylmethyl bedeutet, umsetzt und diese anschließend, wieder bevorzugt in Gegenwart basischer Mittel oder in Form ihrer Salze, mit einer Verbindung der Formel III in 1-Stellung substituiert, wobei Verbindungen der Formel I e (s. Patentanspruch 6) erhalten werden, und jene Verbindungen der Formel I e, in denen $R^6$ eine Benzyl- oder Diphenylmethylgruppe oder aber einen Alkoxymethyl- oder Alkoxyalkoxymethyl-Rest darstellt, unter reduzierenden bzw. hydrolytischen Bedingungen in erfindungsgemäße Verbindungen der Formel I f (s. Patentanspruch 6) überführt, die man gewünschtenfalls nachträglich wieder mit einer Verbindung der Formel III oder IV zu erfindungsgemäßen Verbindungen der Formel Ic bzw. Ie umsetzt.

Eine weitere Ausführungsform d) besteht darin, erfindungsgemäße Verbindungen der Formel Id bzw. Ie, in denen $R^5$ bzw. $R^6$ einen Oxoalkyl-Rest bedeutet, mit üblichen Reduktionsmitteln an der Ketogruppe zu den entsprechenden erfindungsgemäßen hydroxyalkylierten Xanthinen zu reduzieren.

Die hierbei als Ausgangsstoffe verwendeten 3-Alkyl- oder 1,3-Dialkylxanthine der Formel II bzw. V und "Alkylierungsmittel" der Formeln III, IV und IVa sind größtenteils bekannt oder lassen sich nach literaturbekannten Methoden leicht herstellen. So können die tertiären Alkohole der Formel III beispielsweise durch metallorganische Synthese erhalten werden, indem man die sterisch nicht gehinderten Halogenketone der Formel Hal-$(CH_2)_n$—CO—$CH_3$ (VIIa) in einer sogenannten Aufbaureaktion unter reduktiver Alkylierung der Carbonylgruppe mit Alkyl-metall-verbindungen $R^4$-M, worin M Metall, vornehmlich Magnesium, Zink oder Lithium bedeutet, etwa in Form der Alkylmagnesiumhalogenide $R^4$-MgHal (Grignard-Verbindungen) oder der Alkyllithium-Verbindungen $R^4$-Li unter üblichen Bedingungen umsetzt (s z.B. Houben-Weyl, Bd. VI/1 a, Teil 2 (1980), S. 928—40, insbesondere S. 1021 ff. und 1104—1112). Eine gleichartige Umsetzung der Halogenketone gemäß Formel Hal-$(CH_2)_n$-CO—$R^4$ (VIIb) mit Methylmagnesiumhalogeniden oder Methyllithium führt ebenfalls zum Ziel.

Auch die den Formeln VIIa und VIIb entsprechenden Hydroxyketone lassen sich direkt oder unter vorübergehender Maskierung der Hydroxygruppe, etwa durch Acetalisierung, beispielsweise mit 5,6-Dihydro-4H-pyran, glatt mit den Alkyl-metall-verbindungen in üblicher Weise in Diole überführen (s. z.B. Houben-Weyl, Bd. VI/1 a, Teil 2 (1980), S. 1113—1124), aus denen durch selektive Veresterung der endständigen, primären Hydroxylfunktion mit Sulfonsäure- oder Phosphorsäurehalogeniden bzw. -anhydriden, vorteilhaft in Gegenwart basischer Mittel, Verbindungen der Formel III gebildet werden.

Weitere Möglichkeiten zum Aufbau der tertiären Alkoholderivate gemäß Formel III bestehen in der

Monometallierung von ω-Chlor-1-bromalkanen zu ω-Chloralkyl-metall-verbindungen (Houben-Weyl, Bd. XIII/2 a (1973), S. 102 und 319) und deren anschließende Reaktion mit den Ketonen $R^4—CO—CH_3$, wobei das Ausmaß der Nebenprodukt-Bildung aus den intermediär entstehenden Alkanolaten aufgrund ihrer Tendenz zum Ringschluß unter Metallsalz-Eliminierung durch angemessene Temperatursteuerung hintanzuhalten ist, oder in der Verwendung von ω-Halogen-1-alkanolen als Ausgangsstoffe, die man vorzugsweise in Form der Tetrahydropyranyl-(2)-äther oder auch nach mit einer beliebigen Alkyl-metall-verbindung vorgenommenen Alkanolat-Bildung an der Hydroxy-Gruppe ($MO—(CH_2)_n$-Hal) in üblicher Weise metalliert (s. z.B. Houben-Weyl, Bd. XIII/2 a (1973, S. 113), dann mit den Ketonen $R^4—CO—CH_3$ zu den im vorhergehenden Absatz erwähnten Diolen umsetzt (Houben-Weyl, Bd. VI/1 a, Teil 2 (1980), S. 1029) und nachfolgend die primäre Hydroxygruppe mit geeigneten Sulfonsäure- oder Phosphorsäurederivaten selektiv verestert.

Einen bequemen Zugang zu Verbindungen der Formel III, in denen $R^4$ eine Methylgruppe darstellt, bietet auch die Reaktion von ω-Halogenalkansäurealkylestern (Hal-$(CH_2)_n$-COO-Alkyl) mit zwei Äquivalenten einer Methyl-metall-verbindung, wobei der Ester über das Keton zum tertiären Alkohol unter Einführung zweier Methylreste reagiert (Houben-Weyl, Bd. VI/1 a, Teil 2 (1980), S. 1171—1174). In gleicher Weise lassen sich ω-Hydroxy-carbonsäureester ohne und mit Schutz der Hydroxy-Gruppe, beispielsweise in Form der Tetrahydropyranyl-(2)- oder Methoxymethyl-äther oder gegebenenfalls auch der Lactone als cyclische Ester, mit Methyl-metall-Verbindungen in Diole überführen (s. z.B. Houben-Weyl, Bd. VI/1 a, Teil 2 (1980), S. 1174—1179), aus denen wieder- um durch selektive Veresterung der primären Hydroxylfunktion mit Sulfonsäure- oder Phosphorsäurehalogeniden bzw. -anhydriden aktive Alkylierungsmittel der Formel III erhalten werden.

Geeignete, nach den voranstehend beschriebenen Methoden herstellbare Verbindungen der Formel III sind somit die [(ω-1)-Hydroxy-(ω-1)-methyl]-butyl-, -pentyl, -hexyl- und -heptyl-, die [(ω-2)-Hydroxy-(ω-2)-methyl]-pentyl-, -hexyl-, -heptyl- und -octyl- sowie die [(ω-3)-Hydroxy-(ω-3)-methyl]-hexyl-, -heptyl-, -octyl- und -nonyl-chloride, -bromide-, -jodide, -sulfonate und -phosphate.

Unter den zur Einführung von $R^5$ in die 1- oder 7- und von $R^6$ in die 7-Position de Xanthingerüstes geeigneten Verbindungen der Formel $R^5—X$ (IV) bzw. $R^6—X$ (IVa) nehmen die Alkoxymethyl- und Alkoxyalkoxymethyl-Derivate insofern eine Sonderstellung ein, als deren Halogenide zwar erfolgreich als Reaktionsmittel einsetzbar sind, aber zumindest bei großtechnischer Anwendung toxikologische Probleme aufwerfen können. Daher ist in diesem speziellen Fall der Einsatz der entsprechenden Sulfonate zu bevorzugen, die beispielsweise durch Umsetzung von gemischten Anhydriden aliphatischer Carbonsäuren und aliphatischer oder aromatischer Sulfonsäuren (M. H. Karger et al., J. Org. Chem. *36* (1971), S. 528—531) mit Formaldehyd-dialkylacetalen oder-dialkoxyalkyl-acetalen in übersichtlich und nahezu vollständig verlaufender Reaktion leicht zugänglich sind (M. H. Karger et al., J. Amer. Chem. Soc. *91* (1969), S. 5663—5665):

$$R^7{-}SO_2{-}O{-}CO{-}(C_1{-}C_4)Alkyl \;+\; R^8{-}O{-}CH_2{-}O{-}R^8$$

$$\big\downarrow\; -(C_1{-}C_4Alkyl{-}CO_2R^8$$

$$R^7{-}SO_2{-}O{-}CH_2{-}O{-}R^8$$

Hierbei stellen $R^7$ einen aliphatischen Rest, wie Methyl, Äthyl oder Trifluormethyl, oder einen aromatischen. Rest, beispielsweise Phenyl, 4-Tolyl oder 4-Bromphenyl, vorzugsweise aber Methyl oder 4-Tolyl, und $R^8$ eine unter die Definition von $R^5$ bzw. $R^6$ fallende Alkyl- oder Alkoxyalkylgruppe dar.

Die Reaktion kann sowohl in Substanz als auch in einem wasserfreien, gegenüber den Reaktionspartnern interten aprotischen Lösungsmittel bei Temperaturen zwischen −20° und +40°C, vorzugsweise zwischen 0° und 20°C, durchgeführt werden. Eine Zwischenisolierung der hochreaktiven, hydrolyseempfindlichen und hitzelabilen Sulfonate ist nicht erforderlich; sie werden zweckmäßig unmittelbar als Rohprodukte zur Substitution am Stickstoff der Xanthine verwendet, wobei sich der sonst übliche Zusatz eines basischen Kondensationsmittels erübrigt.

Die Umsetzung der mono- oder disubstituierten Xanthinderivate I b, I f, II, V und VI mit den betreffenden Alkylierungsmitteln der Formel III oder IV bzw. IVa erfolgt gewöhnlich in einem gegenüber den Reaktionsteilnehmern inerten Verteilungs oder Lösungsmittel. Als solche kommen vor allem dipolare, aprotische Solventien, beispielsweise Formamid, Dimethylformamid, Dimethylacetamid, N-Methylpyrrolidon, Tetramethylharnstoff, Hexamethylphosphorsäuretriamid, Dimethylsulfoxid, Aceton oder Butanon in Frage; es können aber auch Alkohole, wie Methanol, Äthylenglykol und dessen Mono- bzw. Dialkyläther, wobei die Alkylgruppe 1 bis 4 C-Atome hat, beide zusammen aber höchstens 5 C-Atome haben, Äthanol, Propanol, Isopropanol und die Verschidenen Butanole; Kohlenwasserstoffe, wie Benzol, Toluol oder Xylole; halogenierte Kohlenwasserstoffe, wie Dichlormethan oder Chloroform; Pyridin sowie Mischungen der genannten Lösungsmittel oder deren Gemische mit Wasser Verwendung finden.

Die "Alkylierungsreaktionen" werden zweckmäßig in Gegenwart eines basischen Kondensationsmittels durchgeführt. Hierfür eignen sich beispielsweise Alkali- oder Erdalkalihydroxide, -carbonate, -hydride, -alkoholate und organische Basen, wie Trialkylamine (z.B. Triäthyl- oder Tributylamin), quartäre Ammonium- oder Phosphoniumhydoxide und vernetzte Harze mit fixierten, gegebenenfalls substituierten Ammonium- oder Phosphoniumgruppen. Die Xanthinderivate können aber auch unmittelbar in Form ihrer gesondert hergestellten Salze, etwa der Alkali-, Erdalkali- oder gegebenenfalls substituierten Ammonium- oder Phosphoniumsalze, in der Alkylierungsreaktion einsetzt werden. Weiterhin lassen sich die mono- und disubstituierten Xanthinverbindungen sowohl in Gegenwart der vorgenannten anorganischen Kondensationsmittel als auch in Form ihrer Alkali- oder Erdalkalisalze unter Mithilfe von sogenannten Phasentransferkatalysatoren, beispielsweise tertiären Aminen, quartären Ammonium- oder Phosphoniumsalzen oder auch Kronenäthern, bevorzugt in einem zweiphasigen System unter der Bedingungen einer Phasentransferkatalyse, bequem alkylieren. Geeignete, zumeist kommerziell erhältliche Phasentransferkatalysatoren sind unter anderen Tetra ($C_1$—$C_4$)alkyl und Methyltrioctylammonium- und -phosphonium-, Methyl-, Myristyl-, Phenyl- und Benzyl-tri($C_1$—$C_4$)alkyl- und Cetyltrimethylammonium- sowie ($C_1$—$C_{12}$)Alkyl- und Benzyl-triphenylphosphoniumsalze, wobei sich in der Regel jene Verbindungen, die das größere und symmetrischer gebaute Kation besitzen, als effektiver erweisen.

Bei der Einführung der Reste Ia, $R^5$ und $R^6$ nach den voranstehend beschriebenen Verfahrens weisen arbeitet man in allgemeinen bei einer Reaktionstemperatur zwischen 0°C und dem Sidepunkt des jeweils verwendeten Reaktionsmediums, vorzugsweise zwischen 20° und 130°C, gegebenenfalls bei erhöhtem oder vermindertem Druck, aber gewöhnlich bei Atmosphärendruck, wobei die Reaktionszeit von weniger als einer Stunde bis zu mehreren Stunden betragen kann.

Die Umsetzung der 3-Alkylxanthine II zu den erfindungsgemäßen Verbindungen der Formel Ic erfordert die Einführung zweier tertiärer Hydroxyalkylgruppen. Hierbei können entweder nacheinander gleiche oder verschiedene Substituenten oder auch zwei gleichartige Hydroxyalkylgruppen ohne Isolierung von Zwischenprodukten in einer Eintopfreaktion mit dem Xanthingerüst verknüpft werden.

Die reduktive Abspaltung der Benzyl- und Diphenylmethylgruppe aus Verbindungen der Formel I e unter Bildung der erfindungsgemäßen Xanthinderivate I f, die in 7-Position ein Wasserstoffatom tragen, erfolgt unter Standardbedingungen, die vor allem im Rahmen der Schutzgruppen-Technik bie Alkaloid- und Peptidsynthesen entwickelt wurden und somit als weitgehend bekannt vorausgesetzt werden können. Neben der chemischen Reduktion insbesondere der Benzylverbindungen mit Natrium in flüssigem Ammoniak (Houben-Weyl, Bd. XI/1 (1957), S. 974—975) kommt vorzugsweise die Eliminierung der beiden vorgenannten Aralkylgruppen durch katalytische Hydrogenolyse mit Hilfe eines Edelmetall-Katalysators in Betracht (Houben-Weyl, Bd. XI/1 (1957), S. 968—971 und Bd. IV/1 c, Teil 1 (1980), S. 400—404). Als Reaktionsmedium dient hierbei gewöhnlich ein niederer Alkohol (gegebenenfalls unter Zusatz von Ameisensäure oder auch Ammoniak), ein aprotisches Lösungsmittel wie Dimethylformamid oder insbesondere Eisessig; aber auch deren Gemische mit Wasser können Verwendung finden. Geeignete Hydrierungskatalysatoren sind vornehmlich Palladium-Schwarz und Palladium auf Aktivkohle oder Bariumsulfat, während andere Edelmettale wie Platin, Rhodium und Ruthenium aufgrund konkurrierender Kernhydrierung häufig Anlaß zu Nebenreaktionen geben und deshalb nur bedingt einsetzbar sind. Die Hydrogenolyse wird zweckmäßig bei Temperaturen zwischen 20° und 100°C und unter Atmosphärendruck oder bevorzugt leichtem Überdruck bis zu etwa 10 bar durchgeführt, wobei in der Regel Reaktionszeiten von einigen Minuten bis zu mehreren Stunden benötigt werden.

Die 1,3,7-trisubstituierten Xanthine der Formel I e, die eine Alkoxymethyl- oder Alkoxyalkyloxymethylgruppe in der Position von $R^6$ tragen, stellen O,N-Acetale dar. Demzufolge lassen sich ihre 7-ständigen Substituenten under den üblichen Bedingungen der sauren Hydrolyse abspalten (vergl. (Houben-Weyl, Bd. VI/1 b (1984), S. 741—745), wobei ebenfalls die 7H-Verbindungen der Formel I f gebildet werden. Bevorzugte hydrolytisch eliminierbare Reste sind beispielsweise die Methoxy-, Athoxy- und Propoxymethyl- sowie die Methoxyäthoxy- und Äthoxyäthoxy-methylgruppe. Die Reaktion wird vorteilhaft unter Erwärmen in verdünnten Mineralsäuren wie Salz- oder Schwefelsäure, gegebenenfalls unter Zusatz von Eisessig, Dioxan, Tetrahydrofuran oder einem niederen Alkohol als Lösungsvermittler, durchgeführt. Mitunter kommen auch Perchlorsäure oder organische Säuren, wie Trifluoressig-, Ameisen- und Essigsäure, in Verbindung mit katalytischen Mengen von Mineralsäuren in Frage. Besonders die Alkoxy alkoxymethyl- Verbindungen lassen sich auch mit Hilfe von Lewis-Säuren wie Zinkbromid und Titantetrachlorid in wasserfreiem Milieu, vorzugsweise in Dichlormethan oder Chloroform spalten, wobei die intermediär gebildeten 7-Brommethyl- oder 7-Bromzink-Derivate im Zuge der wäßrigen Aufarbeitung spontan hydrolysieren. Bei der Spaltung in mineralsaurer Lösung ist die Reaktionstemperatur so zu wählen, daß keine merkliche Dehydratisierung der 1-ständigen tertiären Hydroxyalkylgruppe eintritt; sie sollte daher in Regel unter 100°C liegen.

Die Reduktion der in der Position von $R^5$ bze. $R^6$ eine Oxoalkylgruppe tragenden Xanthine der Formeln Id und Ie zu den entsprechenden Hydroxyalkylverbindungen kann zwar im Prinzip sowohl mit unedlen Metallen als auch durch katalytische Hydrierung erfolgen, doch die Methode der Wahl besteht in der unter sehr milden Bedingungen und mit hohen Ausbeuten verlaufenden Umsetzung mit einfachen Metallhydriden ($MH_n$), komplexen Metallhydriden ($M^1[M^2H_n]_m$) oder Organometallhydriden (Houben-Weyl, Bd. VI/1 d (1981), S. 267—282, und Bd. VI/1 b (1984), S. 141—155). Von den zahlreichen zur Reduktion von

Ketonen einsetzbaren komplexen Metallhydriden seien beispielhaft die am häufigsten verwendeten Reagentien genannt, nämlich Lithiumalanat, Lithiumboranat and insbesondere Natriumboranat, das aufgrund seiner geringeren Reaktivität einfacher zu handhaben ist und vor allem das Arbeiten in alkoholischen, alkoholisch wäßrigen und rein wäßrigen Lösungen oder Suspensionen erlaubt. Auch Nitrile wiek Acetonitril können neben den sonst üblichen inerten Lösungsmitteln wie Äthern (z. B. Diäthyläther, Tetrahydrofuran, 1,2-Dimethoxyäthan), Kohlenwasserstoffen und Pyridin als Reaktionsmedium benutzt werden. Die Hydrierung, die zweckmäßig bei Temperaturen zwischen 0°C und dem Siedepunkt des jeweiligen Lösungsmittels, vorzugsweise jedoch bei Raumtemperatur ausgeführt wird, verläuft in der Regel rasch und ist innerhalb einiger Minuten bis weniger Stunden beendet.

Die tertiären Hydroxyalkylxanthine gemäß Formel I lassen sich auch dadurch herstellen, daß man e) substituierte Xanthine der Formel VIII (s. Patentanspruch 6), die

$e_1$) in den Positionen von $R^9$ und $R^{10}$ zwei gleiche oder verschiedene Gruppen der Formel $-(CH_2)_n-CO-CH_3$ (IXa) oder $-(CH_2)_n-CO-R^4$ (IXb) oder aber nur einen Substituenten der Formel IXa oder IXb und in der anderen Stellung Wasserstoff oder den Rest $R^5$ bzw. $R^6$ enthalten, im Falle von IXa mit ($C_1-C_3$)Alkyl- bzw. im Falle von IXb mit Methyl-metall-Verbindungen unter reduktiver "Alkylierung" der Carbonylgruppen zu den erfindungsgemäßen Xanthinen der Formeln Ib bis If umsetzt oder

$e_2$) in den Positionen von $R^9$ und $R^{10}$ zwei gleiche oder verschiedene Gruppen der Formel $-(CH_2)_n-$Hal (X), wobei Hal vorzugsweise Chlor oder Brom bedeutet,

oder nur einen derartigen Rest und Wasserstoff oder den Substituenten $R^5$ bzw. $R^6$ in der anderen Position tragen, endständig metalliert und anschließend mit den Ketonen der Formel $R^4-CO-CH_3$ (XI) unter reduktiver Alkylierung der Carbonylgruppe zu den erfindungsgemäßen Xanthinen der Formeln Ib bis If umsetzt oder

$e_3$) in den Positionen von $R^9$ und/oder $R^{10}$ die Gruppe 5$-(CH_2)_n$-COO$-(C_1-C_4)$Alkyl (XIII) und gegebenenfalls Wasserstoff oder den Rest $R^5$ bzw. $R^6$ in der anderen Position tragen, mittels zweier Äquivalent einer Methyl-metall-verbindung pro Alkoxycarbonylfunktion in solche erfindungsgemäßen Xanthine de Formeln Ib bis If überführt, in denen $R^4$ die Bedeutung von Methyl hat, oder

$e_4$) in den Positionen von $R^9$ und $R^{10}$ zwei gleiche oder verschiedene Reste der Formel

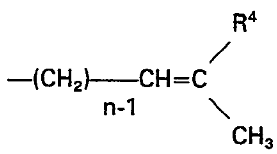

(XIII)

oder nur einen derartigen Rest und in der anderen Stellung Wasserstoff oder den Rest $R^5$ bzw. $R^6$ tragen, wobei, die Gruppe XIII die C=C-Doppelbindung auch in stellungsisomeren Anordnungen am verzweigten, C-Atom, z.B. als

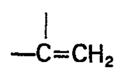

enthalten kann, durch säurekatalysierte, der Markownikoff-Regel gehorchende Hydratisierung in die erfindungsgemäßen Xanthine der Formeln Ib bis If umwandelt und gewünschtenfalls anschließend die nach den Methoden $e_1$) bise $_4$) erhaltenen erfindungsgemäßen tertiären Hydroxyalkylxanthine der Formeln Ib und If die in 1- bzw. 7-Stellung ein Wasserstoffatom tragen, gegebenenfalls in Gegenwart basischer Mittel oder in der Form ihrer Salze, mit den Alkylierungsmitteln der Formel III oder IV bzw. IVa zu den trisubstituierten Verbindungen der Formeln Ic bzw. Id oder Ie umsetzt,

wobei in den voranstehenden Formeln $R^2$, $R^4$, $R^5$, $R^6$ und n die oben angegebenen Bedeutungen haben.

Die hierfür als Ausgangsstoffe benötigten 3-alkylierten Mono- oder Di-oxoalkyl-(VIIIa), -(ω-halogenalkyl)- (VIIIb), -(ω-alkoxycarbonylalkyl)-(VIIIc) und -alkenyl-xanthine (VIIId) sind entweder bekannt oder lassen sich beispielsweise aus den 3-Alkylxanthinen II und den Sulfonyloxy- oder Halogen-ketonen VII a und VII b,-(ω-Halogenalkylsulfonaten oder 1,-(ω-Dihalogenalkanen (vergl. z. B. V. B. Kalcheva et al., Journal für prakt. Chemie *327* (1985), S. 165—168), -(ω-Sulfonyloxy- oder -(ω-Halogen-carbonsäurealkylestern bzw. Sulfonyloxy- oder Halogen-alkenen entsprechend Formel XIII unter den für die Alkylierung von mono- und disubstituierten Xanthinen mit den Verbindungen der Formeln III und IV bereits ausführlich beschriebenen Reaktionsbedingungen leicht herstellen.

Bei den metallorganischen Umsetzungen der in den Resten $R^9$ und $R^{10}$ funktionalisierten Xanthine VIII a bis VIII c wird prinzipiell in der gleichen Weise vorgegangen wie für die Herstellung der als Alkylierungsmittel verwendeten tertiären Alkohole gemäß Formel III geschildert. So kann die reduktive Alkylierung der Ketone VIII a und der Ester VIII c beispielsweise mit Alkyl-kalium-, -natrium-, -lithium-, -magnesium-, -zink-, -cadmium-, -aluminium- und -zinn-Verbindungen erfolgen. Die neuerdings empfohlenen Alkyl-titan- und zirkonium-Verbindungen (D. Seebach et al., Angew. Chem. *95* (1983)l, S. 12—26) sind ebenfalls einsetzbar. Da jedoch die Alkyl-metall-Verbindungen des Natriums und Kaliums aufgrund ihrer hohen Reaktivität zu Nebenreaktionen neigen und die des Zinks und Cadmiums verhältnismäßig reaktionsträge sind, werden gewöhnlich die Alkyl-lithium- und -magnesium(Grignard)-Verbindungen bevorzugt.

Die Stark nucleophilen metallorganischen Verbindungen sind sehr hydrolysen- und oxydationsempfindlich. Ihre sichere Handhabung erfordnet daher ein Arbeiten in wasserfreiem Medium, gegebenenfalls unter Schutzgasatmosphäre. Obliche Lösungs- oder Verteilungsmittel sind vornehmlich jene, die sich auch für die Herstellung der Alkyl-metall-verbindungen eignen. Als solche kommen vor allem Äther mit einem oder mehreren Äthersaurestoffatomen, beispielsweise Diäthyl- Dipropyl-, Dibutyl- oder Diisoamyläther, 1,2-Dimethoxyäthan, Tetrahydrofuran, Dioxan, Tetrahydropyran, Furan und Anisol, und aliphatische oder aromatische Kohlenwasserstoffe, wie Petroläther, Cyclohexan, Benzol, Toluol, Xylole, Diäthylbenzole und Tetrahydronaphthalin in Frage; es können aber auch tertiäre Amine, wie Triäthylamin, oder dipolare, aprotische Solventien, etwa Hexamethylphosphorsäuretriamid, sowie Mischungen der genannten Lösungsmittel mit Erfolg verwendet werden. Bei Umsetzung der Carbonylverbindungen VIII a und VIII c mit den Grignard-Verbindungen der Formel $R^4$-MgHal kann auch vorteilhaft so vorgegangen werden, daß man die metallorganische Verbindung in einem Äther vorlegt und das Keton oder den Ester als Lösung in Dichlormethan oder 1,2-Dichloräthan hinzutropft. Häufig empfiehlt sich ein Zusatz von Magnesiumbromid, das aufgrund seiner Beteiligung an dem komplexartigen cyclischen Obergangszustand die Nucleophilie der metallorganischen Verbindung zu erhöhen vermag.

Die Vereinigung von Keton oder Ester und metallorganischer Verbindung erfolgt in der Regel bei Temperaturen zwischen −20° und 100°C vorzugsweise zwischen 0° und 60° oder bei Raumtemperatur ohne Außenkühlung, wobei man die Alkylmetall-Verbindung üblicherweise in geringfügigem Überschuß anwendet. Anschließend wird die Umsetzung gewöhnlich durch kurzzeitiges Erhitzen unter Rückfluß beendet, wozu im Regelfall Zeitspannen von einigen Minuten bis zu wenigen Stunden ausreichen. Die Zersetzung des gebildeten Alkanolats erfolgt vorzugsweise mit wäßriger Ammoniumchlorid-Lösung oder verdünnter Essigsäure.

Für die Metallierung der ω-Halogenalkylxanthine VIII b kommen vornehmlich metallisches Magnesium und Lithium in Betracht. Demgegenüber spielt de ebenfalls mögliche Austausch des Halogenatoms gegen Lithium mit Hilfe von lithiumorganischen Regentien, meist Butyl-(1)-, Butyl-(2)-, tert.-Butyl- oder Phenyllithium, eine untergeordnete Rolle. Vorrangig bedient man sich jedoch der Grignard-Verbindungen, bei deren Herstellung vorteilhaft in den für die Umsetzung der Xanthine VIII a und VIII c mit Alkyl-metallverbindungen als besonders geeignet aufgezählten Äthern, Kohlenwasserstoffen, tertiären Aminen oder aprotischen Solventien bei Temperaturen zwischen 25° und 125°C, vorzugsweise unter 100°C gearbeitet wird. Führt man die Metallierungsreaktion in Kohlenwasserstoffen aus, so bewährt sich häufig der Zusatz eines Äthers, wie Tetrahydrofuran, oder eines tertiären Amins, wie Triäthylamin, in stöchiometrischer Menge. Auch die Verwendung von Katalysatoren, wie Butanol, Aluminiumchlorid, Siliziumtetrachlorid, Tetrachlormethan und Aluminium- oder Magnesiumalkoholaten, kann förderlich sein. Bei dem Halogen-Metall-Austausch reagieren die Chloride zwar gewöhnlich langsamer als die entsprechenden Bromide und Jodide, liefern dafür aber in der Regel bessere Ausbeuten an metallorganischer Verbindung. Zur Beschleunigung des Reaktionseintritts empfiehlt sich oftmals die Zugabe von etwas Magnesiumbromid, einiger Körnchen Jod oder einiger Tropfen Brom, Tetrachlormethan oder Methyljodid unter leichtem Erwärmen. Die gewonnenen Grignard-Verbindungen werden normalerweise nicht isoliert, sondern unmittelbar mit den Ketonen der Formel XI unter den für die reduktive Alkylierung der Xanthine VIII a und VIII c beschriebenen Reaktionsbedingungen umgesetzt.

Die Wasseraddition and die C—C Doppelbindung der Alkenylxanthine VIII d mit dem Strukturelement der Formel XIII, bei der die Hydroxygruppe nach der Markownikoff-Regel an das wasserstoffärmere C-Atom unter Bildung tertiärer Alkohole tritt, gelingt üblicherweise in wäßriger Lösung oder Suspension in Gegenwart starker Säuren wie Schwefel-, Salpeter- oder Phosphorsäure. Auch Halogenwasserstoff- und Sulfonsäuren, etwa Trifluormethylsulfonsäure, saure Austauschharze, Bortrifluorid-Komplexe oder Oxalsäure können als Katalysatoren verwendet werden. Bevorzugt ist jedoch das Arbeiten in Schwefelsäure, wobei in der Regel eine Säurekonzentration von 50 bis 65% und Temperaturen von 0° bis 10°C ausreichen. Gelegentlich können aber auch niedrigere oder höhere Säurekonzentrationen und/oder Reaktionstemperaturen angebracht sein. Auf jeden Fall sollte die Umsetzungstemperatur so niedrig wie irgend möglich gehalten werden, da sich bei Temperaturen oberhalb von etwa 60°C die rückläufige Dehydratisierung zum Olefin störend bemerkbar machen kann.

Mitunter bietet auch der Zusatzt eines gegenüber Säuren inerten Lösungsmittels wie 1,4-Dioxan, Benzol oder Toluol Vorteile. Da bei der säurekatalysierten Hydratisierung, insbesondere bei Anwendung höherer Säurekonzentrationen, intermediär Ester entstehen können, empfiehlt es sich, den Reaktionsansatz nach de Säureinwirkung zwecks Esterhydrolyse mit viel Wasser unter kurzzeitigem Erwärmen zu behandeln oder aber alkalisch aufzuarbeiten.

Die experimentellen Bedingungen für die wahlfreie Umwandlung der erfindungsgemäßen 1- und 7H-Verbindungen Ib bzw. If in die trisubstituierten Xanthine der Formeln Ic oder Id bzw. Ie durch N-Alkylierung mit den Verbindungen III bzw. IV oder IVa wurden bereits vorn detailliert beschrieben.

In die Verbindungen, in denen einer der Reste $R^1$ und $R^3$ eine Dihydroxyalkylgruppe darstellt, läßt sich diese Dihydroxyalkylgruppe nach üblichen Methoden, wie sie z.B. in der EP—OS 75 850 beschrieben sind, aufbauen bzw. einführen.

Die tertiären Hydroxyalkylxanthine der Formel I können in Abhängigkeit von der Kettenlänge des Alkylrestes $R^4$ (mindesens $C_2$) und/oder der Struktur des Substituenten $R^5$ (z. B. 2-Hydroxypropyl) ein oder zwei asymmetrische C-Atome besitzen und somit in steroisomeren Formen vorliegen. Die Erfindung

betrifft daher sowohl die reinen stereoisomeren Verbindungen als auch deren Gemische.

Die erfindungsgemäßen Xanthinverbindungen der Formel I eignen sich aufgrund ihrer wertvollen pharmakologischen und günstigen metabolischen Eigenschaften, z. B. gegenüber den mischfunktionellen mikrosomalen Oxydasen der Leber, in hervorragender Weise für die Verwendung als Wirkstoffe in Arzneimitteln, insbesondere in solchen, die eine effektivere prophylaktische und kurative Behandlung der durch periphere und cerebrale Durchblutungsstörungen bedingten Erkrankungen, wie der peripheren arteriellen Verschlußkrankheit, ermöglichen und somit eine wesentliche Bereicherung des Arzneimittelschatzes darstellen. Sie können entweder für sich allein, beispielsweise in Form von Mikrokapseln, in Mischungen untereinander oder in Kombination mit geeigneten Trägerstoffen verabreicht werden.

Gegenstand der Erfindung sind folglich auch Arzneimittel, die mindestens eine Verbindung der Formel I als Wirkstoff enthalten.

Die erfindungsgemäßen Arzneimittel werden im allgemeinen oral oder parenteral verabreicht, aber auch eine rektale Anwendung ist prinzipiell möglich. Geeginete feste oder flüssige galenische Zubereitungsformen sind beispielsweise Granulate, Pulver, Tabletten, Dragees, (Mikro)Kapseln, Zäpfchen, Sirupe, Emulsionen, Suspensionen, Aerosole, Tropfen oder injizierbare Lösungen in Ampullenform sowie Präparate mit protrahierter Wirkstoff-Freigabe, bei deren Herstellung üblicherweise Hilfsmittel, wie Trägerstoffe, Spreng-, Binde-, Überzugs-, Quellungs-, Gleit- oder Schmiermittel, Geschmacksstoffe, Süßungsmittel oder Lösungsvermittler, Verwendung finden. Als häufig verwendete Hilfsstoffe seien z. B. Magnesiumcarbonat, Titandioxid, Laktose, Mannit und andere Zucker, Talkum, Milcheiweiß, Gelatine, Stärke, Vitamine, Cellulose und ihre Derivate, tierische und pflanzliche Öle, Polyäthylenglykole und Lösungsmittel, wie etwa steriles Wasser, Alkohole, Glycerin, und mehrwertige Alkohole genannte.

Vorzugsweise werden die pharmazeutischen Präparate in Dosierungseinheiten hergestellt und verabreicht, wobei jede Einheit als aktiven Bestandteil eine bestimmte Dosis einer Verbindung gemäß Formel I enthält. Bei festen Dosierungseinheiten, wie Tabletten, Kapseln und Suppositorien, kann diese Dosis bis zu 100 mg, bevorzugt jedoch 100 bis 600 mg, und bei Injektionslösungen in Ampullenform bis zu 300 mg, vorzugsweise aber 20 bis 200 mg, betragen.

Für die Behandlung eines erwachsenen Patienten sind — je nach Wirksamkeit der Verbindungen gemäß Formel I am Menschen — Tagesdosen von 100 bis 2000 mg Wirkstoff, vorzugsweise 300 bis 900 mg, bei oraler Verabreichung und von 10 bis 500 mg, bevorzugt 20 bis 200 mg, bei intravenöser Applikation indiziert. Unter Umständen können jedoch such höhere oder niedriger Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

Schließlich können die Xanthinderivate der Formel I bei der Herstellung der vorgenannten galenischen Zubereitungsformen auch zusammen mit anderen geeigneten Wirkstoffen, beispielsweise Antithrombotika, Antihyperlipidämika, Analgetika, Sedativa, Antidepressiva, antianginösen Mitteln, Cardiotonika, Antiarrhythmika, Diuretika, Antihypertensiva einschließlich β-Rezeptoren- und Calciumkanal-Blockern, Plasmaexpandern und anderen Vasotherapeutika, formuliert werden.

Die Struktur aller nachstehend beschriebenen Verbindungen wurde durch Elementaranalyse und IR- sowie $^1H$—NMR—Spektren gesichert. Die gemäß den nachfolgenden Beispielen 1 bis 14, 55 und 56 und die auf analoge Weise hergestellten Verbindungen 15 bis 54 der Formel I sind in Tabelle 1 zusammengefaßt. Im folgenden wird unter Äther jeweils Diäthyläther und unter Vakuum das der Wasserstrahlpumpe verstanden.

## Beispiel 1

### 7-(5-Hydroxy-5-methylhexyl)-3-methylxanthin

a) 1-Chlor-5-hydroxy-5-methylhexan

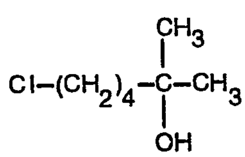

$a_1$) *aus 1-Chlor-5-hexanon:* Zu 44.9 g (0,6 mol) Methylmagnesiumchlorid in Form einer 20%-igen Lösung in Tetrahydrofuran und 200 ml trockenen Äther fügt man unter Rühren bei 0° bis 5°C eine Lösung von 67,3 g (0,5 mol) 1-Chlor-5-hexanon in 50 ml wasserfreiem Äther tropfenweise hinzu. Anschließend wird zunächst eine Stunde lang bei Raumtemperatur und dann für weitere Stunde unter Rückflußkochen nachgerührt, das gebildete tertiäre Alkanolat durch Zugabe 50%-iger wäßriger Ammoniumchlorid-Lösung zersetzt, die Ätherphase abgetrennt und die wäßrige Phase mit Äther ausgeschüttelt. Man wäscht die vereinigten ätherischen Extrakte nacheinander mit wäßriger Natriumhydrogensulfit und Natriumhydrogencarbonat-Lösung sowie wenig Wasser, trocknet über Natriumsulfat, filtriert, engt im

Vakuum ein und unterwirft den flüssigen Rückstand einer fraktionierten Destillation unter vermindertem Druck.

*Ausbeute:*
64,1 g (85,1% der Theorie)
Siedepunkt (20 mbar) 95—97°C
Brechungsindex $n_D^{25}$ = 1,4489
$C_7H_{15}ClO$ (MG = 150,65)

Die Verbindung ließ sich auf analoge Weise auch aus 5-Chlorpentansäuremethyl- oder -äthylester mit der zweifach molaren Menge Methylmagnesiumchlorid herstellen (vergl. Beispiel 13a).

$a_2$) *aus 1-Brom-4-chlorbutan und Aceton:* 24,3 g (1 Grammatom). Magnesium werden nach dem Überschichten mit wasserfreiem Äther mit 10 g 1-Brom-4-chlorbutan versetzt. Sobald die Reaktion angesprungen ist, tropft man weitere 161,5 g Dihalogenalkan (insgesamt 1 mol), gelöst in 200 ml trockenem Äther, so hinzu, daß das Reaktionsgemisch leicht siedet.

Nach beendeter Umsetzung des Metalls erfolgt die tropfenweise Zugabe von 52,3 g (0,9 mol) Aceton, vermischt mit dem gleichen Volumen trockenen Äthers. Nach zweistündigem Nachrühren bei Raumtemperatur wird mit 100 g Eis und gesättigter Ammoniumchlorid-Lösung versetzt, die ätherische Schicht abgetrennt und die wäßrige Phase mehrmals mit Äther extrahiert. Die vereinigten organischen Phasen wäscht man mit wenig Wasser, trocknet über Natriumsulfat, destilliert den Äther im Vakuum ab und fraktioniert den flüssigen Rückstand unter vermindertem Druck.

*Ausbeute:*
71,6 g (52,8% der Theorie)
Siedepunkt (17 mbar) 95°C

b) 7-(5-Hydroxy-5-methylhexyl)-3-methylxanthin

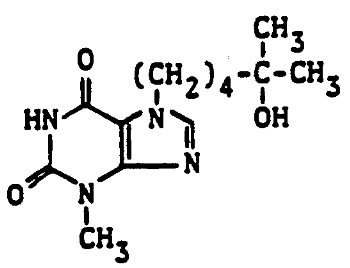

83 g (0,5 mol) 3-Methylxanthin werden in 500 ml 1 N Natronlauge (0,5 mol) heiß gelöst. Man filtriert, destilliert das Wasser unter vermindertem Druck ab und trocknet das zurückbleibende Natriumsalz im Hochvakuum. Nach Zugabe von 1,5 l Dimethylformamid und 75,3 g (0,5 mol) 1-Chlor-5-hydroxy-5-methylhexan wird 6 Stunden unter Rühren bei 100°C erhitzt, heiß filtriert, uner vermindertem Druck eingedampft, der anfallende Rückstand in 1 l heißer 1 N Natronlauge aufgenommen, die heiße Lösung filtriert und nach dem Abkühlen auf Raumtemperatur unter Rühren tropfenweise mit 6 N Salzsäure bis zum Erreichen von pH 9 versetzt. Der Niederschlag wird abgenutscht, neutralgewaschen und im Vakuum getrocknet.

*Ausbeute:*
100,5 g (71,7% der Theorie)
Schmelzpunkt: 228—230°C
$C_{13}H_{20}N_4O_3$ (MG = 280,3)
*Analyse:*

| | | | |
|---|---|---|---|
| Berechnet: | C 55,70% | H 7,19% | N 19,99% |
| Gefunden: | C 55,60% | H 7,31% | N 19,92% |

Beispiel 2

1,7-Bis-(5-hydroxy-5-methylhexyl)-3-methylxanthin

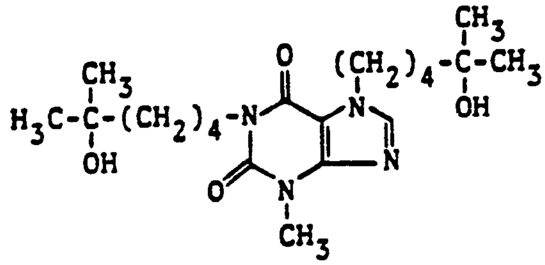

Das Gemisch aus 14 g (0,05 mol) 7-(5-Hydroxy-5-methylhexyl)-3-methylxanthin (Beispiel 1b), 8,2 g (0,054 mol) 1-Chlor-5-hydroxy-5-methylhexan (Beispiel 1a) und 7,5 g (0,054 mol) Kaliumcarbonat in 300 ml Dimethylformamid wird 18 Stunden bei 110°C gerührt, anschließend heiß filtriert und unter vermindertem Druck eingeengt. Man nimmt den Rückstand in Chloroform auf, wäscht zunächst mit verdünnter Natronlauge und dann mit Wasser neutral, trocknet über Natriumsulfat und destilliert das Lösungsmittel im Vakuum ab.

Das Rohprodukt läßt sich vorteilhaft durch Filtration über eine Kieselgel-Säule im Lösungsmittelgemisch Chloroform/Methanol (10:1) und nachfolgendes Ausrühren in Diisopropyläther analysenrein gewinnen.

*Ausbeute:*
14,9 g (75,5% der Theorie)
Schmelzpunkt: 93—95°C
$C_{20}H_{34}N_4O_4$ (MG = 394,5)

*Analyse:*

| | | | |
|---|---|---|---|
| Berechnet: | C 60,89% | H 8,69% | N 14,20% |
| Gefunden: | C 60,89% | H 8,98% | N 14,17% |

Zu derselben Verbindung gelangte man unter anderem auch durch einstufige Dialkylierung von 3-Methylxanthin mit der zweifach molaren Menge an 1-Chlor-5-hydroxy-5-methylhexan oder durch Umsetzung von 1,7-Bis-(5-oxohexyl)-3-methylxanthin mit zwei Äquivalenten Methylmagnesiumchlorid oder -bromid in wasserfreiem Tetrahydrofuran.

Beispiel 3

7-(5-Hydroxy-5-methylhexyl)-3-methyl-1-propylxanthin

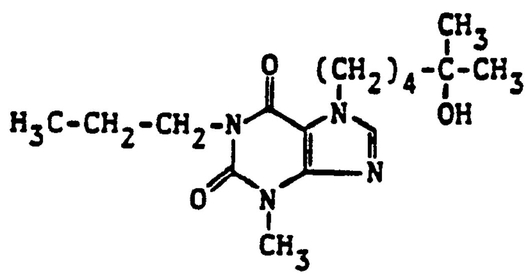

Man erhitzt 40 g (0,14 mol) 7-(5-Hydroxy-5-methylhexyl)3-methylxanthin (Beispiel 1 b) zusammen mit 18, 5 g (0,15 mol) 1-Brompropan und 20,7 g (0,15 mol) Kaliumcarnbonat in 300 ml Dimethylformamid unter Rühren 8 Stunden lang auf 130°C. Nach dem Abkühlen und Einengen bei vermindertem Druck wird mit verdünnter Natronlauge versetzt und mit Chloroform sorgfältig extrahiert Die organische Phase liefert nach dem Neutralwaschen mit Wasser, Trocknen über Natriumsulfat und Eindampfen unter vermindertem Druck ein öliges Rohprodukt, das einfachheitshalber durch Filtration über eine Kieselgel-Säule im Lösungsmittelgemisch Chloroform/Methanol (25:1) und Ausrühren in Diisopropyläther gereinigt wird.

*Ausbeute:*
36,5 g (80,9% der Theorie)
Schmelzpunkt: 59—60°C
$C_{16}H_{26}N_4O_3$ (MG = 322,4)

*Analyse:*

| | | | |
|---|---|---|---|
| Berechnet: | C 59,61% | H 8,13% | N 17,38% |
| Gefunden: | C 59,43% | H 8,01% | N 17,29% |

Sowohl die Alkylierung von 3-Methyl-1-propylxanthin mit 1-Chlor-5-hydroxy-5-methylhexan analog dem nachstehenden Beispiel 4 als auch die Grignard-Synthese mit 3-Methyl-7-(5-oxohexyl)-1-propylxanthin und Methylmagnesiumbromid oder -chlorid in wasserfreiem Tetrahydrofuran führten zu derselben Verbindung.

Beispiel 4
7-(5-Hydroxy-5-methylhexyl)-1,3-dimethylxanthin

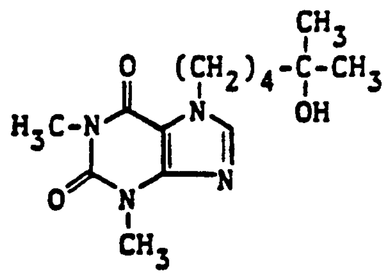

21,8 g (0,1 mol) 1,3-Dimethylxanthin als Kaliumsalz (hergestellt analog Beispiel 1b aus 1,3-Dimethylxanthin und einer äquimolaren Menge Kaliumhydroxid in Wasser) werden mit 16,6 g (0,11 mol) 1-Chlor-5-hydroxy-5-methylhexan des Beispiels 1 in 500 ml Dimethylformamid 18 Stunden lang bei 120°C gerührt. Man läßt erkalten, engt im Vakuum ein, fügt 4 N Natronlauge hinzu und extrahiert das Produkt mit Chloroform. Der mit Wasser neutralgewaschene und getrocknete Extrakt wird unter vermindertem Druck eingedampft und der Rückstand aus Isopropanol/Äther umkristallisiert.

*Ausbeute:*
20,7 g (70,3% der Theorie)
Schmelzpunkt: 106—107°C
$C_{14}H_{22}N_4O_3$ (MG = 294,36)
*Analyse:*

| | | | |
|---|---|---|---|
| Berechnet: | C 57,13% | H 7,53% | N 19,03% |
| Gefunden: | C 57,39% | H 7,67% | N 19,28% |

Alternativ ließ sich die Verbindung unter anderem aus 7-(5-Hydroxy-5-methylhexyl)-3-methylxanthin (Beispiel 1b) und Jodmethan analog Beispiel 3, aus 1,3-Dimethyl-7-(5-oxohexyl)-xanthin und Methylmagnesiumchlorid oder -bromid im wasserfreiem Äther analog Biespiel 9 und aus 1,3-Dimethyl-7-(5-methyl-4-hexenyl)-xanthin durch säurekatalysierte Hydratisierung analog Beispiel 14 herstellen.

Beispiel 5
7-Äthoxymethyl-1-(5-hydroxy-5-methylhexyl)-3-methylxanthin

a) 7-Äthoxymethyl-3-methylxanthin

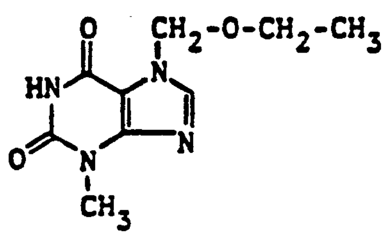

$a_1$) *mit Äthoxymethylchlorid:* 83 g (0,5 mol) 3-Methylxanthin werden in einer Lösung von 20 g (0,5 mol) Natriumhydroxid in 400 ml Wasser heiß gelöst. Nach Filtration engt man im Vakuum ein, destilliert mehrmals Methanol über und trocknet das Natriumsalz im Hochvakuum.

Das trockene Salz wird in 1,3 l Dimethylformamid suspendiert, unter Rühren mit 47,3 g (0,5 mol) Äthoxymethylchlorid versetzt und 18 Stunden bei 110°C gerührt. Anschließend wird heiß filtriert, im Vakuum eingedampft, der Rückstand in 500 ml 2 N Natronlauge gelöst und zur Entfernung von als Nebenprodukt gebildeten 1,7-dialkylierten 3-Methylxanthin mit Chloroform ausgeschüttelt. Die alkalische wäßrige Lösung bringt man mit 2 N Salzsäure unter Rühren auf pH 9, nutscht das gebildete Kristallisat ab,

wäscht zunächst mit Wasser chlorid-frei und dann mit Methanol und trocknet im Vakuum.

*Ausbeute:*
77,6 g (69,2% der Theorie)
Schmelzpunkt: 263—264°C
$C_9H_{12}N_4O_3$ (MG = 224,2)

$a_2$) *mit Äthoxymethyl-4-toluolsulfonat* (ausgehend von 4-toluolsulfonsäurechlorid und Natriumacetat): Man löst 104,9 g (0,55 mol) 4-Toluolsulfochlorid in 100 ml Dimethylformamid und trägt unter Rühren und Eiskühlung 45,1 g (0,55 mol) wasserfreies Natriumacetat ein.

Nach einstündigem Nachrühren bei Raumtemperatur werden 78,1 g (0,75 mol) Formaldehyddiäthylacetal hinzugetropft. Man rührt wiederum eine Stunde bei Raumtemperatur und gibt dann 83 g (0,5 mol) 3-Methylxanthin hinzu. Danach wird das Reaktionsgemisch ohne Zusatz eines basischen Kondensationsmittels für zwei Stunden auf 90°C erhitzt, abgekühlt, das ausgefallene Produkt kalt abgenutscht, mit wenig kaltem Dimethylformamid nachgewaschen, mit Wasser chloridfrei gewaschen, mit Methanol nachgespült und aus Dimethylformamid umkristallisiert.

*Ausbeute:*
98,1 g (87,5% der Theorie)
Schmelzpunkt: 265°C

$a_3$) *mit Äthoxymethyl-4-toluolsulfonat* (ausgehend von 4-Toluolsulfonsäure und Acetanhydrid): Unter Rühren und Kühlen werden 226 g (1,2 mol) 4-Toluolsulfonsäuremonohydrat in 450 g (4,4 mol) Essigsäureanhydrid gelöst und anschließend 30 Minuten auf 70°C erwärmt. Man distilliert die gebildete Essigsäure und überschüssiges Acetanhydrid unter vermindertem Druck ab, verdünnt mit 100 ml Toluol und rührt die erhaltene Lösung unter Kühlung so in 450 ml Dimethylformamid ein, daß die Innentemperatur 20°C nicht übersteigt. Nach tropfenweiser Zugabe von 230 g (2,2 mol) Formaldehyddiäthylacetal und einstündigem Nachrühren bei 20°C werden 166,1 g (1 mol) 3-Methylxanthin hinzugesetzt. Man erwärmt, rührt das Reaktionsgemisch eine Stunde lang bei 100°C, kühlt dann ab, nutscht das ausgefallene Produkt ab, wäscht nacheinander mit je 250 ml Dimethylformamid, Wasser und Methanol und kristalliert aus Dimethylformamid um.

*Ausbeute:*
201 g (89,7% der Theorie)
Schmelzpunkt: 264—265°C

b) 7-Äthoxymethyl-1-(5-hydroxy-5-methylhexyl)-3-methylxanthin

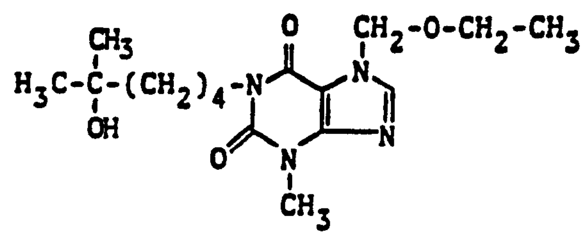

11,2 g (0,05 mol) 7-Äthoxymethyl-3-methylxanthin werden in 300 ml Dimethylformamid mit 7,5 g (0,054 mol) Kaliumcarbonat und 8,2 g (0,054 mol) 1-Chlor-5-hydroxy-5-methylhexan (Beispiel 1 a) versetzt und unter Rühren 5 Stunden auf 110°C erhitzt. Man saugt heiß ab, engt im Vakuum ein, nimmt den Rückstand in Chloroform auf, wächst zunächst mit 1 N Natronlauge und dann mit Waser neutral, trocknet über Natriumsulfat, destilliert das Lösungsmittel unter vermindertem Druck ab und kristallisiert den Rückstand aus Diisopropyläther unter Zusatz von Essigsäureäthylester und Petroläther um.

*Ausbeute:*
14,1 g (83,3% der Theorie)
Schmelzpunkt: 102—103°C
$C_{16}H_{26}N_4O_4$ (MG = 338,4)
*Analyse:*
Berechnet: C 56,79% H 7,74% N 16,56%
Gefunden: C 56,76% H 7,82% N 16,59%

Die Verbindung wurde z.B. auch durch Grignard-Synthese aus 7-Äthoxymethyl-3-methyl-1-(5-oxohexyl)-xanthin mit Methylmagnesiumchlorid in wasserfreiem Äther analog Beispiel 9 erhalten.

Beispiel 6

1-(5-Hydroxy-5-methylhexyl)-3-methylxanthin

a) durch katalytische Hydrogenolyse aus 7-Benzyl-1-(5-hydroxy-5-methylhexyl)-3-methylxanthin:

7-Benzyl-3-methylxanthin

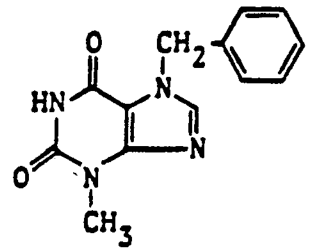

Zu einer Suspension von 83 g (0,5 mol) 3-Methylxanthin in 500 ml Methanol gibt man 20 g (0,5 mol) in 200 ml Wasser gelöstes Natriumhydroxid und rührt eine Stunde bei 70°C, versetzt dann bei derselben Temperatur tropfenweise mit 85,5 g (0,5 mol) Benzylbromid und hält das Reaktionsgemisch für 5 Stunden zwischen 70° und 80°C. Anschließend wird abgekühlt, kalt abgenutscht, das Produkt auf der Nutsche mit Wasser gewaschen, in 1000 ml 1 N Natronlauge heiß gelöst, filtriert und mit 4 Salzsäure unter Rühren langsam auf pH 9,5 gebracht. Man filtriert das Kristallisat von der noch warmen Lösung ab, wäscht mit Wasser chloridfrei und trocknet im Vakuum.

*Ausbeute:*
81,7 g (63,8% der Theorie)
Schmelzpunkt: 262—264°C
$C_{13}H_{12}N_4O_2$ (MG = 256,2)

7-Benzyl-1-(5-hydroxy-5-methylhexyl)-3-methylxanthin

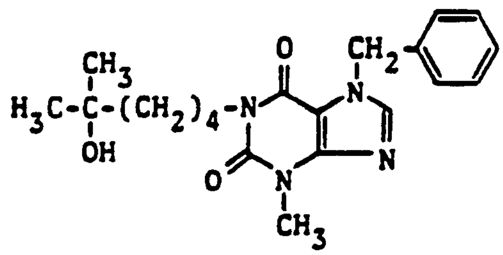

Das Gemisch aus 20,5 g (0.08 mol) 7-Benzyl-3-methylxanthin, 12,4 g (0,09 mol) Kaliumcarbonat und 13,6 g (0.09 mol) des tertiären Alkohols aus Beispiel 1a in 300 ml Dimethylformamid wird 8 Stunden unter Rühren auf 110° bis 120°C erhitzt, anschließend heiß filtriert und unter vermindertem Druck eingedampft. Mann nimmt den Rückstand mit Chloroform auf, wäscht zuerst mit 1 N Natronlauge, dann mit Wasser neutral, trocknet, destilliert das Lösungsmittel im Vakuum ab und kristallisiert den festen Rückstand aus Essigsäureäthylester unter Zusatz von Petroläther um.

*Ausbeute:*
23,8 g (80,3% der Theorie)
Schmelzpunkt: 109—111°C
$C_{20}H_{26}N_4O_3$ (MG = 370,5)

*Analyse:*

| | | | |
|---|---|---|---|
| Berechnet: | C 64,84% | H 7,07% | N 15,12% |
| Gefunden: | C 65,00% | H 7,21% | N 15,24% |

Die Verbindung war unter anderem auch dadurch zugänglich, daß man 7-Benzyl-3-methylxanthin zunächst mit 1-Chlor-5-hexanon unter den voranstehend beschriebenen Reaktionsbedingungen zum 7-Benzyl-3-methyl-1-(5-oxohexyl)-xanthin umsetzte (Ausbeute 90,4% der Theorie, Schmelzpunkt: 82—84°C) und anschließend die Oxohexyl-Seitenkette mit Methylmagnesiumchlorid in wasserfreiem Äther analog Beispiel 9 reduktiv methylierte (Ausbeute: 60,2% der Theorie; Schmelzpunkt: 108—110°C).

1-(5-Hydroxy-5-methylhexyl)-3-methylxanthin

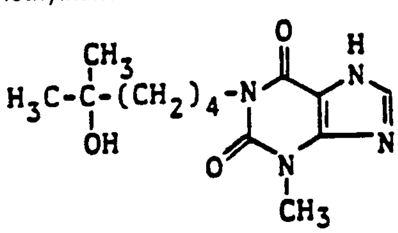

14,l8 g (0,04 mol) des vorgenannten 7-Benzylxanthins werden in 200 ml Eisessig über 1,5 g Palladium (5%) auf Aktivkohle bei 60°C und 3,5 bar innerhalb 24 Stunden unter Schütteln hydriert. Nach dem Abkükhlen überlagert man mit Stickstoff, filtriert den Katalysator ab, engt unter vermindertem Druck ein und kristallisiert den festen Rückstand aus Essigsäureäthylester um.

*Ausbeute:*
95,6 g (85,6% der Theorie)
Schmelzpunkt: 192—193°C
$C_{13}H_{20}N_4O_3$ (MG = 280,3)
*Analyse:*

| | | | |
|---|---|---|---|
| Berechnet: | C 55,70% | H 7,19% | N 19,19% |
| Gefunden: | C 55,63% | H 7,30% | N 20,00% |

b) *durch hydrolytische Dealkoxymethylierung* aus 7-Äthoxymethyl-1-(5-hydroxy-5-methylhexyl)-3-methylxanthin des Beispiels 5b: Man erwärmt 13,5 g (0.04 mol) de Xanthinverbindung des Beispiels 5 b in einem Gemisch aus 300 ml 1 N Salzsäure und 30 ml Eisessig 2,5 Stunden lang unter Rühren auf 70°C, neutralisiert nach dem Abkühlen mit 4 N Natronlauge und extrahiert das Produkt mit Chloroform Der Chloroformextrakt wird getrocknet, im Vakuum zur Trockne gebracht und der Rückstand nach Filtration über eine Kieselgel-Säule im Fließmittel Chloroform/Methanol (10:1) aus Essigsäureäthylester umkristallisiert.

*Ausbeute:*
7,7 g (68,7% der Theorie)
Schmelzpunkt: 191—192°C

Bei gleichartiger hydrolytischer Abspaltung des Propoxymethyl-Restes aus 1-(5-Hydroxy-5-methylhexyl)-3-methyl-7-propoxymethylxanthin (Beispiel 34) wurde die 7H-Verbindung in 75%-iger Ausbeute erhalten.

Beispiel 7
1-(5-Hydroxy-5-methylhexyl)-3-methyl-7-(2-oxopropyl)-xanthin

a) 3-Methyl-7-(2-oxopropyl)-xanthin

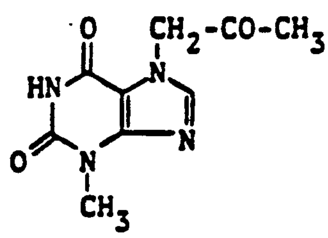

166 g (1 mol) 3-Methylxanthin und 110 g (1,3 mol) Natriumhyrogencarbonat werden in 500 ml Dimethylformamid suspendiert, unter Rühren auf 100°C erwärmt und tropfenweise über einen Zeitraum von zwei Stunden mit 111 g (1,2 mol Chloraceton versetzt. Anschließend wird zwei Stunden bei 100°C nachgerührt, dann abgekühlt und der gebildete Niederschlag abgenutscht und fünfmal je 50 ml Dimethylformamid gewaschen. Nach Aufnahme des Produktes in 60°C warme 1 N Natronlauge versetzt man mit verdünnter Salzsäure bis pH 9, saugt heiß ab, wäscht mit Wasser chloridfrei, spült mit Methanol nach und trocknet bei 80°C im Trockenschrank.

*Ausbeute:*
190 g (85,5% der Theorie)
Schmelzpunkt: 300°C
$C_9H_{10}N_4O_3$ (MG = 222,2)

Verwendeteman Natrium- oder Kaliumcarbonat als basisches Kondensationsmittel oder setzteman das Natrium- oder Kaliumsalz des 3-Methylxanthins ein, so lagen die Ausbeuten wesentlichen niedriger (bei höchstens 70%)

b) 1-(5-Hydroxy-5-methylhexyl)-3-methyl-7-(2-oxopropyl)-xanthin

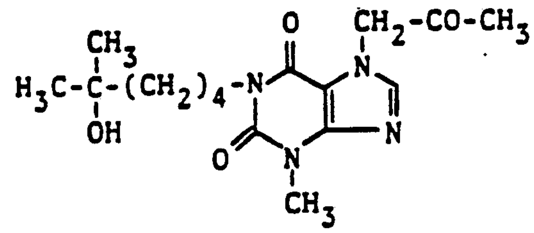

22,2 g (0,1 mol) des Xanthins aus Stufe a) werden mit 16,6 g (0,11 mol) 1-Chlor-5-hydroxy-5-methylhexan (Beispiel 1a) und 15,2 g (0,11 mol) Kaliumcarbonat in 500 ml Dimethylformamid unter den bei Beispiel 2 beschriebenen Versuchsbedingungen umgesetzt und aufgearbeitet. Das durch Säulenchromatographie gereinigte Reaktionsprodukt wird abschließend aus Diisopropyläther unter Zusatz von Essigsäureäthylester in der Siedehitze umkristallisiert.

*Ausbeute:*
26,7 g (79,4% der Theorie)
Schmelzpunkt: 78—80°C
$C_{16}H_{24}N_4O_4$ (MG = 336,4)

*Analyse:*

| | | | |
|---|---|---|---|
| Berechnet: | C 57,13% | H 7,19% | N 16,66% |
| Gefunden: | C 56,85% | H 7,28% | N 16,41% |

Beispiel 8

1-(5-Hydroxy-5-methylhexyl)-7-(2-hydroxypropyl)-3-methylxanthin

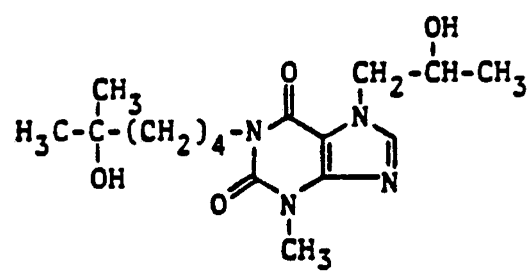

Einer Suspension von 16, 8 g (0,05 mol) 1-(5-Hydroxy-5-methylhexyl)-3-methyl-7-(2-oxopropyl)-xanthin (Beispiel 7b) in 200 ml Methanol werden unter Rühren bei Raumtemperatur 0,95 g (0,025 mol) Natriumboranat zugesetzt. Nach einstündigem Rühren hat sich eine kalare Lösung gebildet. Man zersetzt das überschüssige Hydrid durch Zugabe von 1 ml Eisessig, dampft unter vermindertem Druck ein, nimmt den Rückstand in Chloroform auf, wäscht nacheinander mit verdünnter Natronlauge und Wasser, trocknet über Natriumsulfat und engt im Vakuum zur Trockne ein.

Das feste Rohprodukt wird aus Essigsäureäthylester umkristallisiert.

*Ausbeute:*
15,3 g (90,4% der Theorie)
Schmelzpunkt: 119—120°C
$C_{16}H_{26}N_4O_4$ (MG = 338,4)

*Analyse:*

| | | | |
|---|---|---|---|
| Berechnet: | C 56,79% | H 7,74% | N 16,56% |
| Gefunden: | C 56,52% | H 7,86% | N 16,47% |

Die Verbindung ließ sich auch ausgehend von 3-Methylxanthin in zweistufiger Reaktionsfolge aufbauen, indem man zuerst mit 1-Chlor-2-propanol in 7-Position die 2-Hydroxypropyl-Gruppe einführte (Schmelzpunkt: 278—280°C; Ausbeute: 69,6% der Theorie) und anschließend mit 1-Chlor-5-hydroxy-5-methylhexan (Beispiel 1a) in 1-Stellung alkylierte (Ausbeute: (67,5% der Theorie).

Beispiel 9

1-(5-Hydroxy-5-methylhexyl)-3-methyl-7-propylxanthin

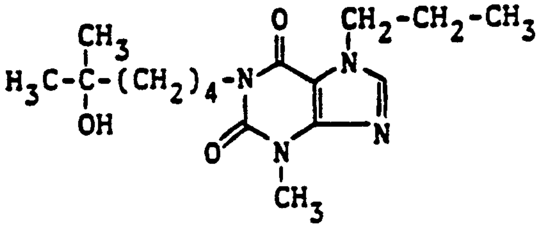

Zu einer Suspension von 61,3 g (0,2 mol) 3-Methyl-1-(5-oxohexyl)-7-propylxanthin in 2 l wasserfreien Äthers fügt man unter kräftigem Rühren bei Raumtemperatur 22,4 g (0,3 mol) Methylmagnesiumchlorid in Form einer 20%-igen Lösung in Tetrahydrofuran tropfenweise hinzu, wobei die Innentemperatur bis auf ca. 30°C ensteigt. Anschließend wird 2 Stunden unter Rühren und Rückfluß erwärmt, zur Zerlegung des gebildeten Alkanolats mit gesättigter wäßriger Ammoniumchlorid-Lösung versetzt, die organische Phase abgetrennt und zweimal mit je 500 ml Wasser gewaschen. Die gesammelten Wasserphasen werden nochmals gründlich mit Dichlormethan extrahiert. Man vereinigt den Dichlormethan-Extrakt mit der ätherischen Phase, trocknet über Natriumsulfat, filtriert und dampft unter vermindertem Druck ein, wobei 59,0 g Rohprodukt (91,5% der Theorie) erhalten werden, die man durch Umkristallisation aus Diisopropyläther reinigt.

*Ausbeute:*
49,8 g (77,2% der Theorie)
Schmelzpunkt: 81—82°C
$C_{16}H_{26}N_4O_3$ (MG = 322,4)
*Analyse:*

| | | | |
|---|---|---|---|
| Berechnet: | C 59,61% | H 8,13% | N 17,38% |
| Gefunden: | C 59,72% | H 8,09% | N 17,44% |

Günstiger gestaltet sich der Reaktionsablauf, wenn man die Grignard-Lösung vorlegt, mit 200 ml wasserfreien Äthers verdünnt, das 3-Methyl-1-(5-oxohexyl)-7-propylxanthin, gelöst in 300 ml trockenen Dichlormethans, unter Rühren bei 10° bis 15°C hinzutropft und eine Stunde bei Raumtemperatur nachrührt, wobei sich das Keton vollständig umsetzt. Man fügt die wäßrige Ammoniumchlorid-Lösung hinzu, destilliert die organischen Lösungsmittel unter vermindertem Druck ab und extrahiert den tertiären Alkohol mit Chloroform. Die Ausbeute an Reinprodukt beträgt 92,1% der Theorie.

Zu derselben Verbindung gelangteman unter anderem durch Alkylierung der Verbindung des Beispiels 6 mit 1-Brom- oder 1-Chlorpropan analog Beispiel 3, durch Umsetzung von 3-Methyl-7-propylxanthin mit dem tertiären Alkohol des Beispiels 1a analog Beispiel 2 oder durch säurekatalysierte Hydratisierung von 3-Methyl-1-(5-methyl-4-hexenyl)-7-propylxanthin analog dem untenstehenden Beispiel 14.

Beispiel 10

1-(5-Hydroxy-5-methylheptyl)-3,7-dimethylxanthin

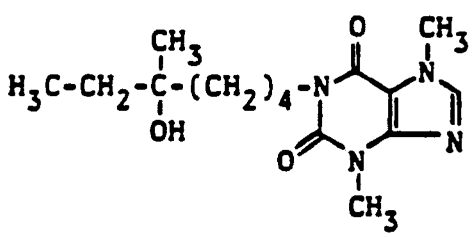

20,0 g (0,15 mol) Äthylmagnesiumbromid als 40%-ige Lösung in Äther werden vorgelegt und 27,8 g (0,1 mol) 3,7-Dimethyl-1-(5-oxohexyl)-xanthin in 1 l trockenen Athers unter Rühren bei Raumtemperatur tropfenweise zudosiert, wobei ein voluminöser Niederschlag entsteht. Der Ansatz wird erwärmt und eine Stunde lang unter leichtem Rückflußkochen nachgerührt.

Anschließend arbeitet man, wie im voranstehenden Beispiel 9 beschrieben, auf und erhält 25 g öliges Rohprodukt (81,1% der Theorie), das allmählich durchkristallisiert und durch Umlösen aus Diisopropyläther unter Zusatz von etwas Essigsäureäthylester in der Siedehitze gereinigt wird.

*Ausbeute:*
22,9 g (74,3% der Theorie)
Schmelzpunkt: 83—84°C
$C_{15}H_{24}N_4O_3$ (MG = 308,4)
*Analyse:*

| | | | |
|---|---|---|---|
| Berechnet: | C 58,42% | H 7,84% | N 18,17% |
| Gefunden: | C 58,33% | H 8,02% | N 18,21% |

Beispiel 11

1-(5-Hydroxy-5-methylhexyl)-7-(2-hydroxy-2-methylpropyl)-3-methylxanthin

a) 3-Methyl-1-(5-oxohexyl)-7-(2-oxopropyl)-xanthin

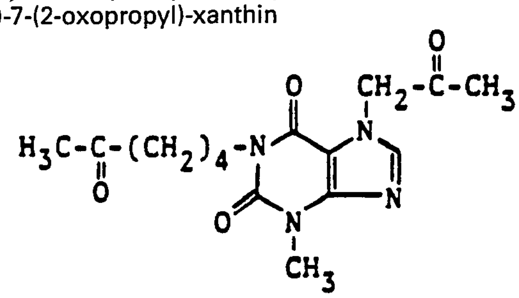

22,2 g (0,1 mol) 3-Methyl-7-(2-oxopropyl)-xanthin aus Beispiel 7a werden zusammen mit 14,8 g (0,11 mol) 1-Chlor-5-hexanon und 15,2 g (0,11 mol) Kaliumcarbonat in 500 ml Dimethylformamid 1,5 Stunden bei 110°C gerührt. Anschließend läßt man unter weiterem Rühren langsam erkalten, filtriert, wäscht das Salz auf der Nutsche mit Dimethylformamid gründlich nach, engt die Lösung unter vermindertem Druck ein, nimmt mit 200 ml Methanol auf, setzt 50 ml Wasser und 2 ml konzentrierte Schwefelsäure hinzu und kocht eine Stunde unter Rückfluß. Nach Entfernen des Methanols im Vakuum wird mit 33 %iger Natronlauge alkalisiert und sorgfältig mit Chloroform ausgeschüttelt.

Die vereinigten Chloroformextrakte wäscht man mit wenig Wasser neutral, trocknet über Natriumsulfat und destilliert das Lösungsmittel im Vakuum ab, wobei 25,8 g festes Rohprodukt (80,5% der Theorie) anfallen, das aus Äthanol unter Zusatz von Petroläther in der Siedehitze umkristallisiert wird.

*Ausbeute:*
23,1 g (72,1% der Theorie)
Schmelzpunkt: 111—113°C
$C_{15}H_{20}N_4O_4$ (MG = 320,3)

*Analyse:*

| | | | |
|---|---|---|---|
| Berechnet: | C 56,24% | H 6,29% | N 17,49% |
| Gefunden: | C 56,31% | H 6,35% | N 17,21% |

b) 1-(5-Hydroxy-5-methylhexyl)-7-(2-hydroxy-2-methylpropyl)-3-methylxanthin

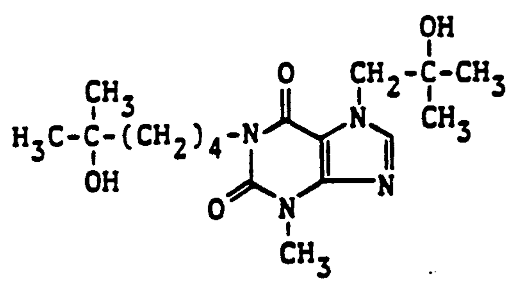

In 22,4 g (0,3 mol) Methylmagnesiumchlorid in Form der käuflichen 20%-igen Lösung in Tetrahydrofuran fügt man unter Feuchtigkeitsausschluß und kräftigem Rühren bei Raumtemperatur 32,0 g (0,1 mol) des zweifach oxoalkylierten Xanthins aus Stufe a) in 100 ml wasserfreien Dichlormethans langsam hinzu. Nach beendeter Zugabe wird erwärmt und der Reaktionsgemisch 2 Stunden lang bei Rückflußtemperatur gehalten, bevor man gemäß Bespiel 9 aufarbeitet und das Rohprodukt aus Essigsäureäthylester umkristallisiert.

*Ausbeute:*
25,8 g (73,2% der Theorie)
Schmelzpunkt: 121—123°C
$C_{17}H_{28}N_4O_4$ (MG = 352,4)

*Analyse:*

| | | | |
|---|---|---|---|
| Berechnet: | C 57,93% | H 8,01% | N 15,90% |
| Gefunden: | C 57,70% | H 7,93% | N 15,83% |

Die Verbindung wurde unter anderem auch durch zweistufige Synthese aus 3-Methylxanthin erhalten, das man zunächst mit 1-Chlor-2-hydroxy-2-methylpropan zu 7-(2-Hydroxy-2-methylpropyl)-3-methylxanthin umsetzte (Schmelzpunkt: 268—269°C; Ausbeute: 51% der Theorie) und anschließend analog Beispiel 5b mit 1-Chlor-5-hydroxy-5-methylhexan aus Beispiel 1a in 1-Position zum Endprodukt alkylierte (Ausbeute: 79,5% der Theorie).

Beispiel 12

1-(4-Hydroxy-4-methylpentyl)-3-methyl-7-propylxanthin

a) 1-Chlor-4-hydroxy-4-methylpentan

44,9 g (0,6 mol) Methylmagnesiumchlorid, 20%-ig gelöst in Tetrahydrofuran, werden in wasserfreiem Äther mit 60,3 g (0,5 mol) 1-Chlor-4-pentanon gemäß Beispiel $1a_1$ umgesetzt und aufgearbeitet.

*Ausbeute:*
42,7 g (62,5% der Theorie)
Siedepunkt (17 mbar) 77—78°C
$C_6H_{13}ClO$ (MG = 136,6)

b) 1-(4-Hydroxy-4-methylpentyl)-3-methyl-7-propylxanthin

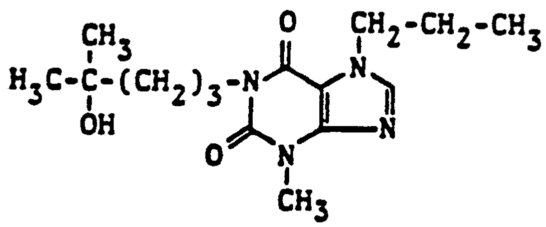

Zu einer Suspension von 20,8 g (0,1 mol) 3-Methyl-7-propylxanthin in 250 ml Methanol gibt man die Lösung von 5,6 g (0,1 mol) Kaliumhydroxid in 100 ml Methanol. Beim Erhitzen entsteht eine klare Lösung, die man unter vermindertem Druck zur Trockne eindampft. Das zurückbleibende Kaliumsalz der Xanthinverbindung wird nach scharfem Trocknen in Hochvakuum mit 500 Dimethylformamid und 15,0 g (0,11 mol) des tertiären Alkohols aus Stufe a) versetzt und 18 Stunden bei 80°C gerührt. Die Aufarbeitung des Ansatzes erfolgt analog Beispiel 5b, wobei 25,1 g Rohprodukt (81,4% der Theorie) erhalten werden, die sich durch Umkrisallisieren aus Diisopropyläther unter zusatz von etwas Eissigsäureäthylester in der Siedehitze reinigen lassen.

*Ausbeute:*
19,2 g (62,3% der Theorie)
Schmelzpunkt: 96—98°C
$C_5H_{24}N_4O_3$ (MG = 308,4)

*Analyse:*

| | | | |
|---|---|---|---|
| Berechnet: | C 58,42% | H 7,84% | N 18,17% |
| Gefunden: | C 58,49% | H 7,82% | N 18,19% |

Weitere gleichberechtigte Darstellungsweisen für diese Verbindung waren die Methylverzweigung der Oxoalkylseitenkette von 3-Methyl-1-(4-oxopentyl)-7-propylxanthin mit Methyllithium oder einem Methylmagnesiumhalogenid analog Beispiel 9, die Alkylierung von 1-(4-Hydroxy-4-methylpentyl)-3-methylxanthin mit 1-Brom- oder 1-Chlorpropan in der 7-Position analog Beispiel 3 und die säurekatalysierte Wasseraddition an die olefinische Doppelbindung von 3-Methyl-1-(4-methyl-3-pententyl)-7-propylxanthin gemäß dem untenstehenden Beispiel 14.

Beispiel 13

3-Äthyl-1-(6-hydroxy-6-methylheptyl)-7-methylxanthin

a) 1-Brom-6-hydroxy-6-methylheptan

89,8 g (1,2 mol) Methylmagnesiumchlorid in Form einer 20%-igen Lösung in Tetrahydrofuran werden zusammen mit 500 ml wasserfreiem Äther vorgelegt und unter Rühren bei 0° bis 5°C tropfenweise mit einer Lösung von 102,3 g (0,46 mol) 6-Bromhexansäureäthylester in 100 ml trockenem Äther versetzt. Danach wird 30 Minuten bei Raumtemperatur und 2 Stunden unter Rückflußkochen nachgerührt, der Ansatz auf Eis gegeben und 50%-ige Wäßrige Ammoniumchlorid-Lösung bis zum vollständigen Wiederauflösen des gebildeten Niederschlages hinzufügt. Man extrahiert mehrmals mit Äther, wäscht den ätherischen Auszug nacheinander mit wäßriger Natriumhydrogensulfit- und Natriumhydrogencarbonat-Lösung sowie Wasser, trocknet über Natriumsulfat, filtriert und entfernt das Lösungsmittel im Vakuum. Der Rückstand wird fraktioniert destilliert.

*Ausbeute:*
80,2 g (83,4% der Theorie)
Siedepunkt (2 mbar) 77—79°C
$C_8H_{17}OBr$ (MG = 209,1)

b) 3-Äthyl-1-(6-hydroxy-6-methylheptyl)-7-methylxanthin

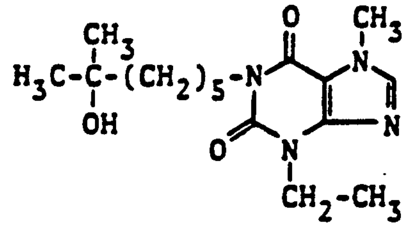

23,2 g (0,1 mol) 3-Äthyl-7-methylxanthin-Kaliumsalz (hergestellt analog Beispiel 12b) werden nach Versetzen mit 500 ml Dimethylformamid und 23,0 g (0,11 mol) des tertiären Bromalkohols aus Stufe a) 8 Stunden unter Rühren auf 120°C erhitzt. Anschließend arbeitet man entsprechend Beispiel 5b auf, wobei ein öliges Produkt anfällt, das nach längerem Stehen durchkristallisiert und aus Diisopropyläther umgelöst wird.

*Ausbeute:*
23,7 g (73,5% der Theorie)
Schmelzpunkt: (86—87°C)
$C_{16}N_{26}N_4O_3$ (MG = 322,4)
*Analyse:*
Berechnet: C 59,61% H 8,13% N 17,38%
Gefunden: C 59,68% H 8,16% N 17,54%

Zu derselben Verbindung gelangte man auch durch Umsetzung von 3-Äthyl-7-methyl-1-(6-oxoheptyl)-xanthin mit Methylmagnesiumchlorid oder -bromid analog Beispiel 9, Methylierung von 3-Äthyl-1-(6-hydroxy-6-methylheptyl)-xanthin mit Methylbromid, Methyljodid, einem Methylsulfonat oder Dimethylsulfat gemäß Beispiel 3 und Wasseranlagerung an die olefinische Doppelbindung von 3-Äthyl-7-methyl-1(6-methyl-5-heptenyl)-xanthin entsprechend dem folgenden Beispiel 14.

Beispiel 14
1-(5-Hydroxy-5-methylhexyl)-3,7-dimethylxanthin

a) 3,7-Dimethyl-1-(5-methyl-4-hexenyl)-xanthin

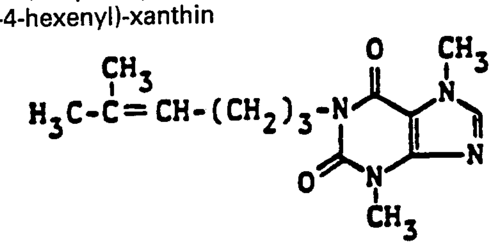

9,0 g (0,05 mol) 3,7-Dimethylxanthin, 8,0 g (0,06 mol) Kaliumcarbonat werden 22 Stunden bei 110°C in 200 ml Dimethylformamid gerührt.
Nach Entfernen des Lösungsmittels im Vakuum versetzt man mit 100 ml 1 N Natronlauge und extrahiert gründlich mit Dichlormethan. Der Extrakt wird nochmals mit verdünnter Natronlauge ausgeschüttelt, mit Wasser neutralgewaschen, getrocknet, unter vermindertem Druck eingedampft und der Rückstand aus Diisopropyläther umkristallisiert.

*Ausbeute:*
10,1 g (73,1% der Theorie)
Schmelzpunkt: 73—75°C
$C_{14}H_{20}N_4O_2$ (MG = 276,3)
*Analyse:*
Berechnet: C 60,85% H 7,30% N 20,27%
Gefunden: C 60,60% H 7,24% N 20,32%

b) 1-(5-Hydroxy-5-methylhexyl)-3,7-dimethylxanthin

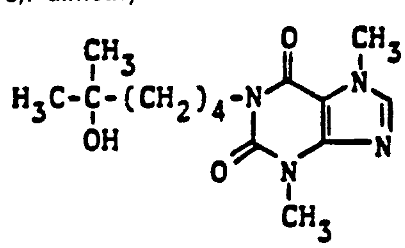

Eine Lösung von 9,5 g (0,034 mol) des Xanthins aus Stufe a) in 50 ml Dioxan und 50 ml 50%-ige Schwefelsäure wird 24 Stunden bei 10°C gerührt. Danach stellt man das Reaktionsgemisch unter Eiskühlung mit 2 N Natronlauge alkalisch und extrahiert das Produkt sorgfältig mit Dichlormethan. Der Auszug wird nacheinander mit 1 N Natronlauge und Wasser gewaschen, getrocknet und unter verminderten Druck eingedampft. Der Rückstand läßt sich durch Umkristallisieren aus einem Gemisch von Diisopropyläther und Isopropanol reinigen.

*Ausbeute:*
7,8 g (77,9% der Theorie)
Schmelzpunkt: 120—121°C
$C_{14}H_{22}N_4O_3$ (MG = 294,4)
*Analyse:*

| | | | |
|---|---|---|---|
| Berechnet: | C 57,13% | H 7,53% | N 19,03% |
| Gefunden: | C 57,21% | H 7,74% | N 18,78% |

Alternativ war die Verbindung unter anderem aus 3,7-Dimethylxanthin und 1-Chlor-5-hydroxy-5-methylhexan analog Beispiel 2 oder 4, aus 3,7-Dimethyl-1-(5-oxohexyl)xanthin und Methylmagnesiumchlorid oder- bromid analog Beispiel 9 und aus der Verbindung des Beispiels 6 und einem Methylierungsmittel analog Beispiel 3 herstellbar.

Beispiel 55

7-(3,4-Dihydroxybutyl)-1-(5-hydroxy-5-methylhexyl)-3-methylxanthin

a) 7-(3-Butenyl)-3-methylxanthin

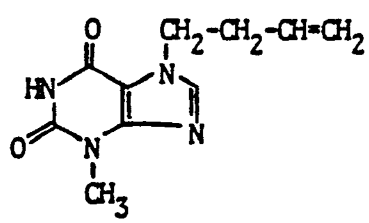

47 g (0,25 mol) 3-Methylxanthin-mononatriumsalz (hergestellt wie in Beispiel 1b beschrieben) und 34,8 g (0,25 mol) 97%iges 1-Brom-3-buten werden in 750 ml Dimethylformamid 8 Stunden lang bei 110°C gerührt. Anschließend entfernt man das ausgefallene Natriumbromid durch Filtration des noch heißen Reaktionsgemisches, dampft das Filtrat im Vakuum ein, löst den festen Rückstand in 250 ml 2 N Natronlauge, erwärmt auf ca. 65°C und versetzt unter Rühren mit 2 N Salzsäure bis pH 9. Nach dem Erkalten wird der gebildete Feststoff abgenutscht, mit Wasser salzfrei gewaschen und nach dem Spülen mit Methanol im Vakuum getrocknet.

*Ausbeute:* 32 g (58,1% der Theorie)
Schmelzpunkt: 242—245°C
$C_{10}H_{12}N_4O_2$ (MG = 220,2)

b) 7-(3-Butenyl)-1-(5-hydroxy-5-methylhexyl)-3-methylxanthin

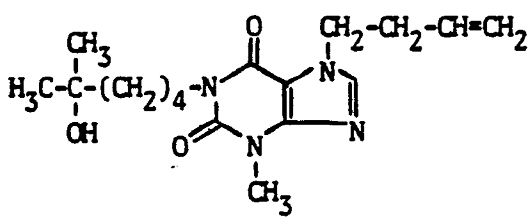

22 g (0,1 mol) des Xanthins aus Stufe a) werden mit 16,6 g (0,11 mol) 1-Chlor-5-hydroxy-5-methylhexan (Beispiel 1a) und 15,2 g (0,11 mol) Kaliumcarbonat in 500 ml Dimethylformamid unter den bei Beispiel 2 beschriebenen Versuchsbedingungen umgesetzt und aufgearbeitet. Das Reaktionsprodukt wird ohne vorherige Säulenchromatographie durch einmalige Umkristallisation aus Essigsäureäthylester unter Zusatz von Petroläther in der Siedehitze rein erhalten.

*Ausbeute:* 25,7 g (76,9 % der Theorie)
Schmelzpunkt: 105—107°C
$C_{17}H_{26}N_4O_3$ (MG = 334,4)

c) 7-(3,4-Epoxybutyl-1-(5-hydroxy-5-methylhexyl)-3-methylxanthin

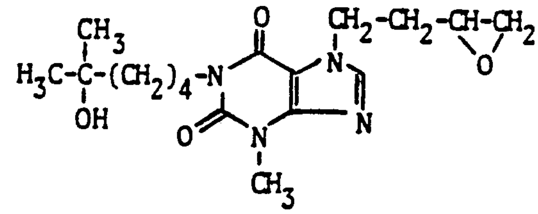

Man trägt in die Lösung von 22 g (0,066 mol) des Xanthins aus Stufe a) in 250 ml Chloroform unter Stickstoffatmosphäre und Rühren bei Raumtemperatur innerhalb von ca. 15 Minuten 15,8 g (0,078 mol) 85%ige 3-Chlorperbenzoesäure ein. Nach 48-stündigem Rühren bei Raumtemperatur wird nacheinander mit 10%iger Natriumdithionit-Lösung, 10%iger Natriumhydrogencarbonat-Lösung und Wasser gewaschen, getrocknet und im Vakuum eingedampft, wobei das Epoxid in nahezu quantitativer Ausbeute als öliges Produkt ($C_{17}H_{26}N_4O_4$; MG = 350,4) anfällt, das unmittelbar in der nachfolgenden Reaktionsstufe d) eingesetzt werden kann.

d) 7-(3,4-Dihydroxybutyl)-1-(5-hydroxy-5-methylhexyl)-3-methylxanthin

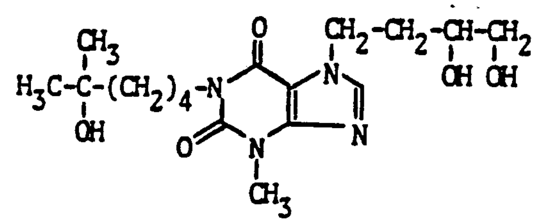

Die Lösung von 23 g (0,065 mol) der Verbindung aus Stufe c) in einem Gemisch aus 120 ml Tetrahydrofuran und 80 ml Wasser wird unter Rürhen bei Raumtemperatur mit 0,4 ml Perchlorsäure (70%ig) versetzt. Nach 5-tägigem Rühren bei Raumtemperatur neturalisiert man mit gesättigter Natriumhydrogencarbonat-Lösung, dampft die Reaktionsmischung im Vakuum ein, nimmt den Rückstand in Chloroform auf und reinigt durch Säulenchromatographie an Kieselgel mit einem Gemisch aus Chloroform/Methanol (Volumenverhältnis 10:1) als Laufmittel.

*Ausbeute:* 19,4 g (81% der Theorie)
Schmelzpunkt: 116—118°C
$C_{17}H_{28}N_4O_5$ (MG = 368,4)
*Analyse:*

| | | | |
|---|---|---|---|
| Berechnet: | C 55,42% | H 7,66% | N 15,21% |
| Gefunden: | C 55,13% | H 7,84% | N 14,98% |

Beispiel 56

7-(2,3-Dihydroxypropyl)-1-(5-hydroxy-5-methylhexyl)-3-methylxanthin

a) 7-(2,3-Dihydroxypropyl)-3-methylxanthin

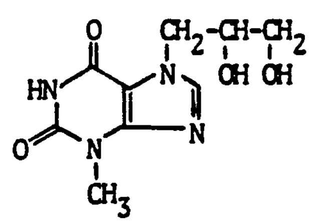

83 g (0,5 mol) 3-Methylxanthin werden in 1250 ml Dimethylformamid gelöst und unter Rühren bei Raumtemperatur portionsweise mit 12 g (0,5 mol) Natriumhydrid versetzt. Man rührt 30 Minuten nach, tropft anschließend 55,3 g (0,5 mol) 1-Chlor-2,3-propandiol in 100 ml Dimethylformamid hinzu und erhitzt das Gemisch 18 Stunden lang unter Rühren auf 110°C. Die Aufarbeitung erfolgt wie bei Beispiel 55 a) beschrieben.

*Ausbeute:* 66,6 g (55,5% der Theorie)
Schmelzpunkt: 302—304°C
$C_9H_{12}N_4O_4$ (MG = 240,2)

b) 7-(2,3-Dihydroxypropyl)-1-(5-hydroxy-5-methylhexyl)-3-methylxanthin

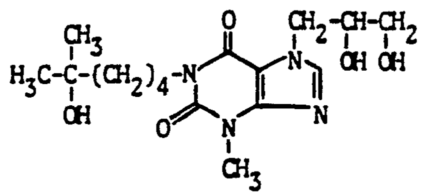

Eine Mischung aus 18 g (0,075 mol) der Xanthinverbindung aus Stufe a), 12,5 g (0,083 mol) 1-Chlor-5-hydroxy-5-methylhexan (Beispiel 1 a) und 11,5 g (0.083 mol) Kaliumcarbonat in 500 ml Dimethylformamid wird 18 Stunden bei 110°C gerührt, anschließend heiß filtriert und im Vakuum eingedampft. Das ölige Rohprodukt, das allmählich durchkristallisiert, läßt sich vorteilhaft durch Filtration über eine Keiselgel-Säule im Lösungsmittelgemisch Chloroform/Methanol (10:1) und abschließende Umkristallisation aus Essigsäureäthylester unter Zusatz von Petroläther in der Siedehitze reinigen.

*Ausbeute:* 15,2 g (57,2% der Theorie)

Schmelzpunkt: 105—107°C

$C_{16}H_{26}N_4O_5$ (MG = 354,4)

*Analyse:*

| | | | |
|---|---|---|---|
| Berechnet: | C 54,22% | H 7,39% | N 15,81% |
| Gefunden: | C 53,87; | H 7,47% | N 15,71% |

Tabelle 1: Verbindungen gemäß Formel I

| Bei= spiel | $R^1$ | $R^2$ | $R^3$ | Schmelz-punkt °C |
|---|---|---|---|---|
| 1 | -H | $-CH_3$ | 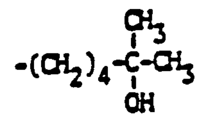 | 228 - 230 |
| 2 | 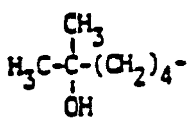 | $-CH_3$ | 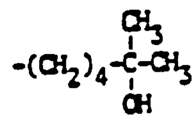 | 93 - 95 |
| 3 | $-C_3H_7$ | $-CH_3$ | 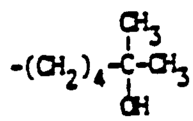 | 59 - 60 |
| 4 | $-CH_3$ | $-CH_3$ | 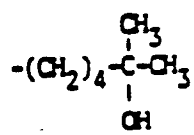 | 106 - 107 |
| 5 | 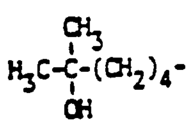 | $-CH_3$ | $-CH_2-O-C_2H_5$ | 102 - 103 |
| 6 | 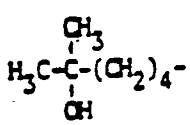 | $-CH_3$ | -H | 192 - 193 |
| 7 | 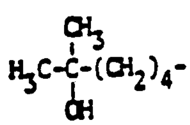 | $-CH_3$ | 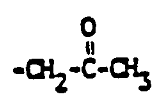 | 78 - 80 |

| Bei= spiel | $R^1$ | $R^2$ | $R^3$ | Schmelz-punkt °C |
|---|---|---|---|---|
| 8 | 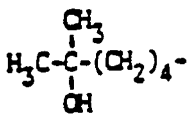 | $-CH_3$ | 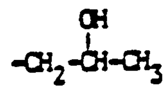 | 119 - 120 |
| 9 | 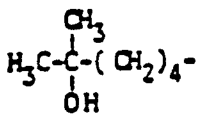 | $-CH_3$ | $-C_3H_7$ | 81 - 82 |
| 10 | 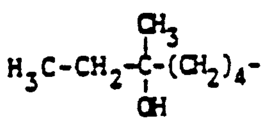 | $-CH_3$ | $-CH_3$ | 83 - 84 |
| 11 | 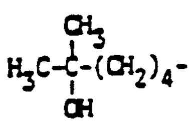 | $-CH_3$ | 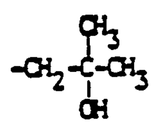 | 121 - 123 |
| 12 | 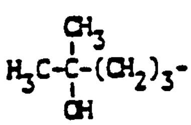 | $-CH_3$ | $-C_3H_7$ | 96 - 98 |
| 13 | 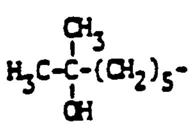 | $-C_2H_5$ | $-CH_3$ | 86 - 87 |
| 14 | 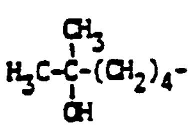 | $-CH_3$ | $-CH_3$ | 120 - 121 |
| 15 | 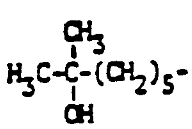 | $-CH_3$ | $-CH_3$ | 112 - 113 |

| Bei= spiel | $R^1$ | $R^2$ | $R^3$ | Schmelz- punkt °C |
|---|---|---|---|---|
| 16 | 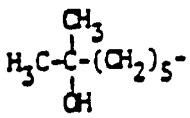 | $-CH_3$ | $-C_3H_7$ | 92 - 94 |
| 17 | 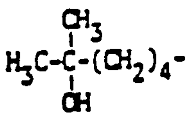 | $-C_2H_5$ | $-CH_3$ | 98 |
| 18 | 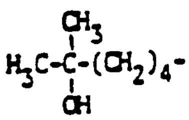 | $-C_2H_5$ | $-C_3H_7$ | 69 - 71 |
| 19 | 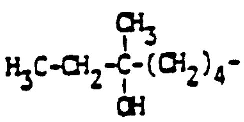 | $-CH_3$ | $-C_3H_7$ | 86 - 87 |
| 20 | 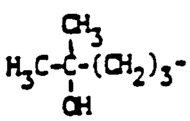 | $-CH_3$ | $-CH_3$ | 117 - 118 |
| 21 | 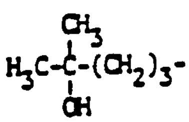 | $-C_2H_5$ | $-CH_3$ | 94 - 95 |
| 22 | 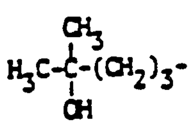 | $-C_2H_5$ | $-C_3H_7$ | 92 - 93 |
| 23 | 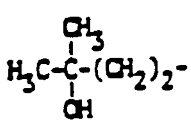 | $-CH_3$ | $-CH_3$ | 146 - 147 |

| Bei=spiel | $R^1$ | $R^2$ | $R^3$ | Schmelz-punkt °C |
|---|---|---|---|---|
| 24 | 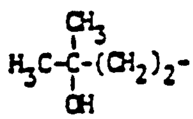 | $-C_2H_5$ | $-CH_3$ | 122 |
| 25 | 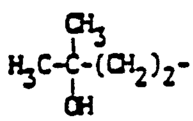 | $-CH_3$ | $-C_3H_7$ | 135 - 137 |
| 26 | $-C_4H_9$ | $-C_4H_9$ | 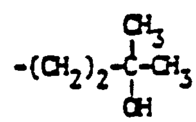 | 69 - 70 |
| 27 | 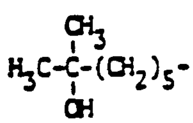 | $-CH_3$ | $-CH_2-O-CH_3$ | 52 - 54 |
| 28 | 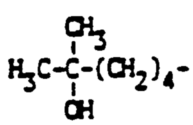 | $-CH_3$ | $-CH_2-O-CH_3$ | 92 - 94 |
| 29 | 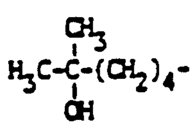 | $-C_2H_5$ | $-CH_2-O-CH_3$ | 61 - 63 |
| 30 | 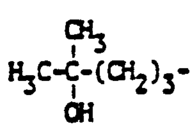 | $-CH_3$ | $-CH_2-O-CH_3$ | 99 - 101 |
| 31 | 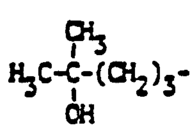 | $-C_2H_5$ | $-CH_2-O-CH_3$ | 94 - 96 |

| Bei= spiel | $R^1$ | $R^2$ | $R^3$ | Schmelz-punkt °C |
|---|---|---|---|---|
| 32 | 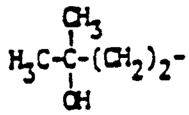 | $-CH_3$ | $-CH_2-O-CH_3$ | 105 - 107 |
| 33 | 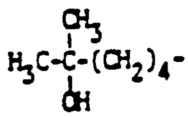 | $-C_2H_5$ | $-CH_2-O-C_2H_5$ | 68 - 70 |
| 34 | 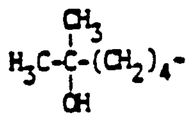 | $-CH_3$ | $-CH_2-O-C_3H_7$ | 83 - 85 |
| 35 | $H_3C-O-(CH_2)_2-$ | $-CH_3$ | 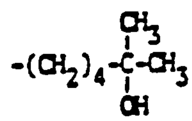 | 63 - 65 |
| 36 | 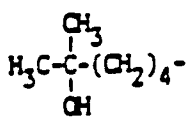 | $-CH_3$ | $-(CH_2)_2-O-CH_3$ | 98 - 99 |
| 37 | 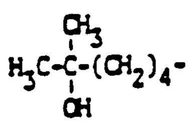 | $-C_2H_5$ | $-(CH_2)_2-O-CH_3$ | 71 |
| 38 | $H_5C_2-O-(CH_2)_2-$ | $-CH_3$ | 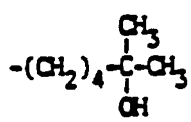 | 77 - 79 |
| 39 | 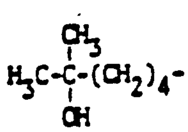 | $-CH_3$ | $-(CH_2)_2-O-C_2H_5$ | 75 |

| Bei= spiel | $R^1$ | $R^2$ | $R^3$ | Schmelz-punkt °C |
|---|---|---|---|---|
| 40 | 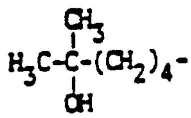 | $-C_2H_5$ | $-(CH_2)_2-O-C_2H_5$ | 52 - 54 |
| 41 | $H_3C-O-(CH_2)_3-$ | $-CH_3$ | 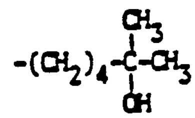 | 45 - 47 |
| 42 | 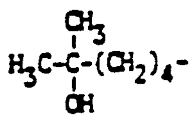 | $-CH_3$ | $-(CH_2)_3-O-CH_3$ | 83 - 84 |
| 43 | 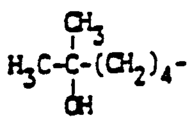 | $-C_2H_5$ | $-(CH_2)_3-O-CH_3$ | 72 - 73 |
| 44 | 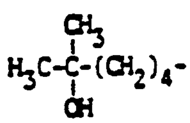 | $-CH_3$ | $-CH_2-O-(CH_2)_2-O-CH_3$ | 88 - 89 |
| 45 | 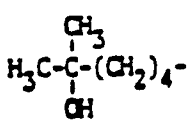 | $-C_2H_5$ | $-CH_2-O-(CH_2)_2-O-CH_3$ | Öl |
| 46 | 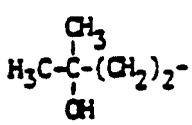 | $-C_4H_9$ | $-CH_2-O-(CH_2)_2-O-CH_3$ | 97 - 98 |
| 47 | 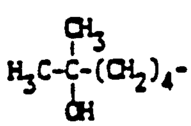 | $-CH_3$ | $-(CH_2)_2-O-(CH_2)_2-OC_2H_5$ | Öl |

| Bei= spiel | $R^1$ | $R^2$ | $R^3$ | Schmelz-punkt °C |
|---|---|---|---|---|
| 48 | 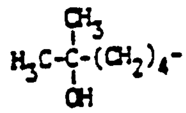 | $-C_2H_5$ | $-(CH_2)_2-O-(CH_2)_2-OC_2H_5$ | Öl |
| 49 | 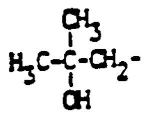 | $-CH_3$ | 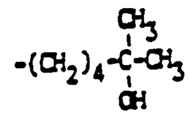 | 65 - 67 |
| 50 | 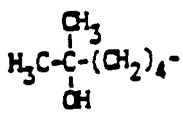 | $-CH_3$ | $-CH_2-CH_2-CH_2-OH$ | 78 - 80 |
| 51 | 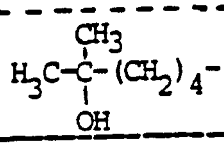 | $-CH_3$ | 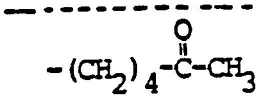 | Öl |
| 52 | " | $-CH_3$ | 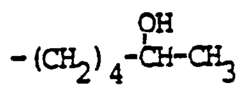 | 69 - 71 |
| 53 | 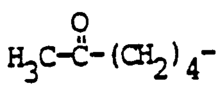 | $-CH_3$ | 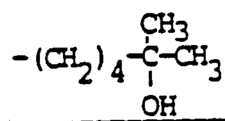 | Öl |
| 54 | 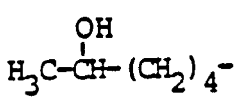 | $-CH_3$ | 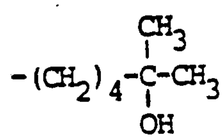 | Öl |
| 55 | 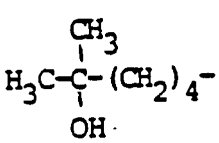 | $-CH_3$ | 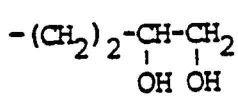 | 116 - 118 |
| 56 | 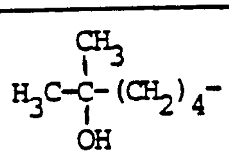 | $-CH_3$ | 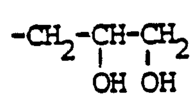 | 105 - 107 |

Pharmakologische Prüfung und Ergebnisse

1. Wirkung auf die periphere arterielle Durchbluntungsstörung

Im letzten Jahrzehnt hat sich in den Vorstellungen über die Pathophysiologie und damit auch in der medikamentösen Therapie der chronischen peripheren arteriellen Verschlußkrankheiten insofern ein beachtlicher Wandel vollzogen, als sich das wissenschaftliche und therapeutische Interesse in zunehmendem Maße von der Makrozirkulation hin zur Mikrozirkulation und hier insbesondere zur kapillären Strombahn verlagerte, über die die Ernährung des angrenzenden Gewebes durch diffusionsvermittelten Substrataustausch erfolgt. Störungen in der Mikrozirkulation manifestieren sich demzufolge in einer zellulären Unterversorgung mit daraus resultierender Gewebsischamie, so daß eine gezielte Therapie darauf ausgerichtet sein muß, die pathologische Inhomogenität der kapillären nutritiven Durchblutung zu beheben und somit den lokalen Sauerstoffpartialdruck ($pO_2$) im ischämischen Gewebe zu normalisieren.

Die Prüfung der erfindungsgemäßen Verbindungen auf ihre die Gewebsversorgung verbessernde Wirkung erfolgte daher anhand von $pO_2$-Messungen im ischämischen Skelettmuskel mit der von D. W. LÜBBERS (Prog, Resp. Res. *3* (Karger, Basel 1969), S. 136—146) und M. KESSLER (Prog. Resp. Res. *3* (Karger, Basel 1969), S. 147—152 und Anesthesiology *45* (1976), S. 184) beschriebenen Versuchsandordnung, wobei das Standardtherapeutikum Pentoxifyllin als Vergleichspräparat in die Untersuchungen miteinbezogen wurde.

Als Versuchstiere dienten männliche Beagle-Hunde in Natrium-Pentobarbital-Narkose (35 mg/kg i.p.) an deren rechten Hinterextremität die Arteria femoralis und ein bestimmtes Areal der Unterschenkelmuskulatur freipräpariert und an deren linken Hinterextremität die Vena femoralis für die Präparatinfusion und die Arteria femoralis für Blutdruckmessungen freigelegt und kanüliert wurden. Die Tiere wurden durch Gabe von Alcuronimchlorid relaxiert (0,1 mg/kg i.v. und danach alle 30 Minuten 0,05 mg/kg i.p.) und künstlich beatmet, um einerseits Spontankontraktionen des Muskels mit negativer Auswirkung auf die $pO_2$-Messungen zu vermeiden und zum anderen ein gleichmäßiges Zuführen von Atemsauerstoff zu gewährleisten.

Ein weiterer, in die Vena femoralis der rechten Hinterextremität eingebundener Katheter diente zur Überwachung der Laktatkonzentration im venösen Ausfluß. Auf das freigelegte Muskelareal wurde nun eine Mehrdraht-Oberflächenelektrode (Fa. Eschweiler, Kiel) zxur kontinuierlichen Registrierung des $pO_2$ aufgesetzt. Sobald sich die $pO_2$-Kurve stabilisiert hatte, wurde die Arteria femoralis mit Hilfe einer Klemme okkludiert, worauf der $pO_2$ in dem von diesem Gefäß versorgten Muskel rasch abfiel, dann infolge der spontanen Eröffnung von Kollateralgefäßen wieder geringfügig anstieg und sich schließlich auf einem gegenüber dem gesunden Muskel stark erniedrigten Niveau einpendelte. Zu diesem Zeitpukt wurde die Prüfsubstanz in wäßriger Lösung entweder intravenös (i.v.) infundiert (0,6 mg/kg/min) oder in einer Dosis von 25 mg/kg intraduodenal (i.d.) verabreicht und der $pO_2$-Anstieg im ischämischen Muskel messend verfolgt. Jeweils vor und nach Okklusion sowie nach Substanzgabe wurde venöses Blut entnommen, um das ausgewaschene Laktat zu bestimmen und damit den physiologischen Status der Tiere zu kontrollieren. Zusätzlich wurden zu Beginn und am Ende eines jeden Versuches die Gaskonzentrationen ($pO_2$ und $pCO_2$) und der pH-Wert im arteriellen Blut der ischämischen Extremität überprüft.

Als Meßparameter für die Präparatwirkung diente im Einzelversuch der maximale prozontuale $pO_2$-Anstieg nach Substanzgabe während der Gefäßokklusion (n = 2—11). Aus diesen Meßwerten wurde für jede Verbindung ein dimensionsloser Wirksamkietsindex W durch Produktbildung aus der prozentualen Häufigkeit positiver Versuche und dem sich aus den positiven Einzelwerten ergebenden mittleren prozentualen $pO_2$-Anstieg berechnet, der sowohl interindividuelle Unterschiede in der Topologie der Muskelvaskularisierung berücksichtigt als auch Responder und Nicht-Responder gegenüber den Testverbindungen miterfaßt und demzufolge einen verläßlicheren Aktivitätsvergleich unter den einzelnen Präparaten gestattet.

2. Wirkung auf die regionale Gehirndurchblutung

Die Wirkung der erfindungsgemäßen Verbindungen auf die regionale Gehirndurchblutung wurde mit Hilfe der Wärmeleittechnik nach F. A. GIBBS (Proc. Soc. exp. Biol. (N. Y.) *31* (1933), S. 141 ff.), H. HENSEL (Naturwissenschaften *43* (1956), S. 477 ff.) und E. BETZ (Acta Neurol. Scand. *Suppl. 14* (1965), S. 29—37) an Katzen beiderlei Geschlechts in Natrium-Pentobarbital-Narkose (35 mg/kg i.p.) untersucht, wobei wiederum das Standardtherapeutikum Pentoxifyllin für Vergleichszwecke herangezogen wurde. Bei dieser Methode bestimmt man mit einer auf die Hirnoberfläche im Bereich des Gyrus marginalis frontalis aufgesetzten Wärmeleitsonde den Wärmetransport von einem Heizpunkt zu einer benachbarten Temperaturmeßstelle in der Sonde, der der Höhe der Gehirndurchblutung dierekt proportional ist. Die prozentuale Zunahme der Wärmetransportzahl $\lambda$ nach Präparatgabe stellt somit ein Maß für die Durchblutungsverbesserung dar.

Die Verbindungen wurden in wäßriger Lösung intravenös appliziert. Die Dosis betrug 3 mg Testsubstanz pro kg Körpergewicht. Für jedes Prüfpräparat wurden 3 bis 5 Einzelversuche durchgeführt und aus den gewonnenen Meßdaten die mittlere prozentuale Gehirndurchbluntungszunahme ermittelt.

3. Akute Toxizität

Die Bestimmung der $LD_{50}$-Bereiche erfolgte standardgemäß über die innerhalb von 7 Tagen bei NMRI-Mäusen nach einmaliger intravenöser (i.v.) oder intraperitonealer (i.p.) Gabe auftretende Mortalität (NMRI = Naval Medical Research Institute).

Die Ergebnisse dieser Untersuchungen, die die Überlegenheit der erfindungsgemäßen Verbindungen entsprechend Formel I gegenüber dem Standardpräparat Pentoxifyllin eindeutig belegen, sind in den nachfolgenden Tabellen 2 und 3 zusammengefaßt.

Tabelle 2: Wirkung auf die gestörte periphere arterielle Durchblutung im Okklusionsmodell am Hund und akute Toxizität an der Maus

| Verbindung aus Beispiel | Okklusionsmodell | | Toxizität |
|---|---|---|---|
| | Applikationsart i.v.: 0,6 mg/kg/min i.d.: 25 mg/kg | Wirksamkeitsindex W (vgl. Text) | $LD_{50}$ (Maus) in mg/kg |
| 3 | i.v.<br>i.d. | 1650<br>1250 | i.v.: 100 - 200 |
| 4 | i.v. | 1300 | i.v.: 100 - 200 |
| 5 | i.v. (0,3 mg/kg/min)<br>i.d. | 2080<br>2909 | i.v.: 150 - 300<br>i.p.: 300 - 600 |
| 6 | i.d. | 700 | i.v.: > 200 |
| 8 | i.v. | 1400 | i.v.: > 200 |
| 10 | i.d. | 1965 | i.v.: > 200 |
| 12 | i.v.<br>i.d. | 866<br>2337 | i.v.: > 200 |
| 14 | i.v. | 960 | i.v.: > 200 |
| 16 | i.v. | 999 | i.p.: 150 - 300 |
| 17 | i.v. | 1525 | i.v.: > 200 |
| 18 | i.v. | 1578 | i.v.: 100 - 200 |
| 28 | i.d. | 2100 | i.v.: > 200 |
| 32 | i.v.<br>i.d. | 2650<br>566 | i.v.: > 200 |
| 37 | i.v.<br>i.d. | 1400<br>950 | i.v.: > 200 |
| 39 | i.v.<br>i.d. | 1567<br>1996 | i.v.: > 200 |
| 44 | i.v. | 1199 | i.v.: > 200 |
| 50 | i.v.<br>i.d. | 2631<br>733 | i.v.: > 200 |
| Pentoxifyllin | i.v.<br>i.d. | 891<br>643 | i.v.: 187 - 209<br>i.p.: 219 - 259 |

Tabelle 3: Wirkung auf die regionale Gehirndurchblutung narkotisierter Katzen nach intravenöser Gabe von 3 mg/kg und akute Toxizität an der Maus

| Verbindung aus Beispiel | Mittlere Zunahme der Hirndurchblutung als $\overline{\Delta\lambda}$ in % | Toxizität $LD_{50}$ (Maus) in mg/kg |
|---|---|---|
| 3 | 15,5 | i.v.: 100 - 200 |
| 9 | 9,4 | i.v.: 100 - 200 |
| 16 | 25,8 | i.p.: 150 - 300 |
| 17 | 9,4 | i.v.: > 200 |
| 18 | 9,4 | i.v.: 100 - 200 |
| 22 | 12,0 | i.v.: >200 |
| 27 | 10,3 | i.v. > 200 |
| 43 | 19,8 | i.v.: > 200 |
| 48 | 14,6 | i.v.: > 200 |
| Pentoxi-fyllin | 8,6 | i.v. 187 - 209<br>i.p.: 219 - 259 |

Die eindeutige Überlegenheit der erfindungsgemäßen Verbindungen insbesondere gegenüber dem am häufigsten zur Therapie peripherer und cerebraler Durchblutungsstörungen eingesetzten Xanthinderivat, dem Pentoxifyllin, ließ sich auch in weiteren speziellen Versuchen eindrucksvoll bestätigen.

Es ist heute allgemein anerkannt, daß ausschließlich gefäßerweiternd wirkende Pharmaka oder Mittel mit sehr starker vasodilatierender Wirkkomponente für die Behandlung von Mikrozirkulationsstörungen ungeeignet sind, da einerseits die physioloigsche vasodilatorische Reserve im Regelfall bereits vollständig ausgeschöpft ist und zum anderen die Gefahr eines Steal-Phänomens besteht, der eine schädliche Umverteilung des nutritiven Blutflusses in der Mikrozirkulation zu Lasten des bereits unterversorgten kranken Gewebes bedeutet. Es wurde deshalb die Hemmwirkung auf die am isolierten durchströmten Kaninchenohr mit Norfenefrin ausgelöste Gefäßkontraktion untersucht. Hierbei zeigte beispielsweise die Verbindung aus Beispiel 5 bis zu einer Konzentration von 100 µm/ml keine Hemmung der Norfenefrin-Wirkung, während Pentoxifyllin im Konzentrationsbereich von 10 bis 100 µg/ml die durch Norfenefrin kontrahierten Gefäße dosisabhängig dilatierte.

In einem chronischen Versuch an Ratten mit einseitigem Arteria-iliaca-Verschluß ließ sich zeigen, daß die Verbindungen der Formel I den Stoffwechsel im ischämischen Skelettmuskel günstig zu beeinflussen

vermögen. Behandelte man nämlich die Tiere 5 Wochen lang z.B. mit der Verbindung aus Beispiel 5, wobei täglich dreimal jeweils 3 mg/kg intraperitoneal verabreicht wurden, so konnte mit Hilfe histochemischer Färbemethoden nachgewiesen werden, daß sich in den beiden untersuchten Muskeln der ischämischen Extremität (Tibialis anterior und Extensor digitorum longus) der Anteil der oxidativen Fasern signifikant erhöht hat. Im Gegensatz dazu übte Pentoxifyllin bei gleichartiger Versuchsdurchfürung keinen direkten Einfluß auf den Muskelstoffwechsel aus.

Die Überlegenheit der Xanthine gemäß Formel I ergab sich auch aus einem weiteren chronischen Versuch, in dem der Einfluß auf die Kontraktilität des ischämischen Skelettmuskels an Ratten mit ligierter rechter Femoralarterie untersucht wurde. Die Tiere erhielten 20 Tage lang das jeweilige Prüfpräparat in einer täglichen oralen Dosis von 25 mg/kg per Schlundsonde. Danach wurde die Ermüdbarkeit des ischämischen Muskels bei elektrischer Reizung mit ca. 80 Kontraktionen pro Minute über den Abfall der Kontraktionskraft nach 1-, 15- und 45-minütiger Stimulation im Vergleich mit unbehandelten Kontrolltieren bestimmt. Hierbei bewirkte etwa die Verbindung des Beispiels 5 — offenkundig infolge einer Stoffwechseloptimierung — eine signifikante Verbesserung der Muskelleistung in der ischämischen Extremität, wobei sogar normale Kontraktilitätswerte wie im linken gesunden (nicht-ischämischen) Muskel unbehandelter Tiere erreicht wurden. Diese Verbesserung ging einher mit einer Steigerung der mitochondrialen Atmungskontrolle (RCR = Respiratory Control Rate). Pentoxifyllin erwies sich in diesen Versuchen als wirkungslos. Die erfindungsgemäßen Verbindungen sind demnach auch für die Behandlung von Störungen des muskulären Energiestoffwechsels unterschiedlicker Genese, insbesondere mitochondrialer Myopathien, geeignet.

Die erfindungsgemäßen pharmazeutischen Präparate eignen sich für die Anwendung sowohl in der Human- als auch Veterinärmedizin.

**Patentansprüche**

1. Tertiäre Hydroxyalkylxanthine der allgemeinen Formel I,

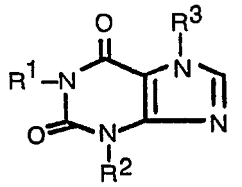

(I)

dadurch gekennzeichnet, daß mindestens einer der Reste $R^1$ und $R^3$ eine tertiäre Hydroxyalkylgruppe der Formel

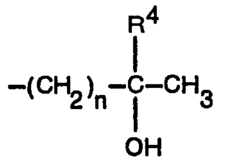

(Ia)

darstellt, in der $R^4$ eine Alkylgruppe mit bis zu 3 C-Atomen und n eine ganze Zahl von 2 bis 5 bedeuten, und — falls nur einer der Reste $R^1$ oder $R^3$ eine solche tertiäre Hydroxyalkylgruppe der Formel Ia bedeutet — der andere Rest für ein Wasserstoffatom oder einen aliphatischen Kohlenwasserstoff-Rest $R^5$ mit bis zu 6 C-Atomen steht, dessen Kohlenstoffkette von bis zu 2 Sauerstoffatomen unterbrochen oder mit einer Oxogruppe oder bis zu zwei Hydroxygruppen substituiert sein kann (wobei die Oxogruppe oder die Hydroxygruppen) durch mindestens 2 C-Atome vom Stickstoff getrennt ist (sind), und $R^2$ eine Alkylgruppe mit 1 bis 4 C-Atomen darstellt.

2. Verbindungen nach Anspruch 1, gekennzeichnet durch wenigstens eines der Merkmale, daß $R^2$ für Methyl oder Äthyl steht, nur einer der beiden Reste $R^1$ oder $R^3$ die im Anspruch 1 definierte tertiäre Hydroxyalkylgruppe darstellt und daß eine im Rest $R^5$ befindliche Hydroxy- oder Oxogruppe durch mindestens zwei C-Atome vom Stickstoff getrennt ist.

3. Verbindungen nach Anspruch 1 oder 2, dadurch gekennzeichnet, daß in Formel I $R^1$ oder $R^3$ [(ω-1)-Hydroxy-(ω-1)-methyl]-pentyl, -hexyl oder -heptyl bedeuten.

4. Verbindungen nach einem oder mehreren der Ansprüche 1 bis 3, dadurch gekennzeichnet, daß in Formel I $R^1$ die tertiäre Hydroxyalkylgruppe darstellt, wobei vorzugsweise $R^1$ [(ω-1)-Hydroxy-(ω-1)-methyl]-pentyl, -hexyl oder -heptyl, $R^2$ Methyl oder Äthyl und $R^3$ Alkyl, Hydroxyalkyl oder Alkoxyalkyl mit jeweils 1 bis 4 C-Atomen bedeuten.

5. Verbindung nach Anspruch 4, dadurch gekennzeichnet, daß sie 7-Äthoxymethyl-1-(5-hydroxy-5-methylhexyl)-3-methylxanthin darstellt.

6. Verfahren zur Herstellung von Xanthinderivaten der Formel I gemäß einem oder mehreren der Ansprüche 1 bis 5, dadurch gekennzeichnet, daß man

a) 3-Alkylxanthine der Formel II,

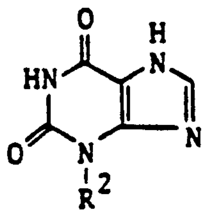

(II)

in der $R^2$ Alkyl mit bis zu 4 C-Atomen darstellt, gegebenenfalls in Gegenwart basischer Mittel oder in Form ihrer Salze mit Alkylierungsmitteln der Formel III,

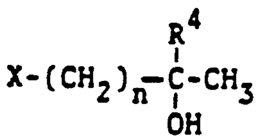

(III)

in der X Halogen oder eine Sulfonsäureester- oder Phosphorsäureester-Gruppierung ist und $R^4$ und n die im Anspruch 1 definierten Bedeutungen haben, zu erfindungsgemäßen Verbindungen der Formel Ib

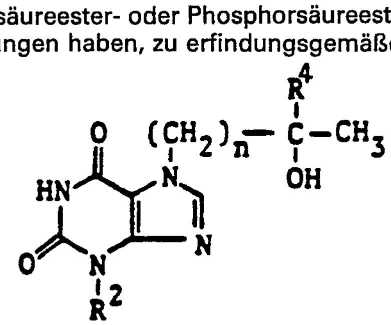

(Ib)

mit einer tertiären Hydroxyalkylgruppe in der Position von $R^3$ und Wasserstoff in der Position von $R^1$ umsetzt, und diese, wiederum bevorzugt in Gegenwart basischer Mittel oder in Form ihrer Salze,

$a_1$) mit demselben oder einem anderen Alkylierungsmittel der Formel III zu erfindungsgemäßen Verbindungen der Formel Ic

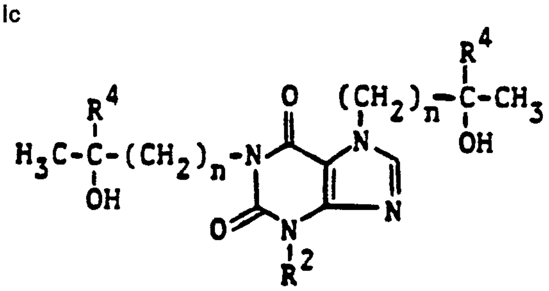

(I c)

mit zwei gleichen oder verschiedenen tertiären Hydroxyalkylgruppen in den Positionen von $R^1$ und $R^3$ alkyliert oder

$a_2$) durch Umsetzung mit einer Verbindung der Formel $R^5$—X (IV), in der X die bei Formel III und $R^5$ die im Anspruch 1 angegebenen Bedeutungen haben, in erfindungsgemäße Verbindungen der Formel Id

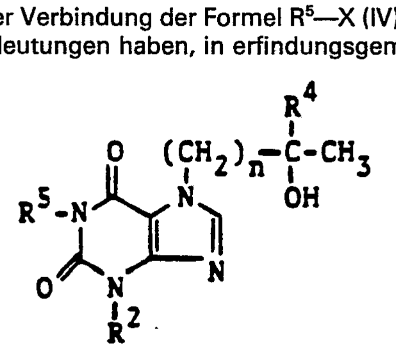

(Id)

umwandelt, oder

b) 1,3-dialkylierte Xanthine der Formel V,

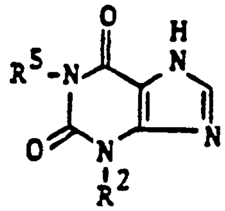

(V)

zweckmäßig in Gegenwart basischer Mittel oder in Form ihrer Salze, durch einstufige Umsetzung mit einer Verbindung der Formel III in 7-Stellung zu Verbindungen der Formel Id substituiert oder

c) die 3-Alkylxanthine der Formel II, ebenfalls vorzugsweise in Gegenwart basischer Mittel oder in Form ihrer Salze, zuerst mit einer Verbindung der Formel $R^6$—X (IVa) unter Bildung von 3,7-disubstituierten Xanthinen der Formel VI,

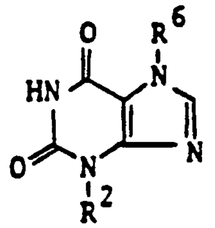

(VI)

wobei $R^6$ die für $R^5$ genannte Bedeutung hat oder Benzyl oder Diphenylmethyl darstellt und X die bei Formel III angegebene Bedeutung hat, umsetzt und diese anschließend, wieder bevorzugt in Gegenwart basischer Mittel oder in Form ihrer Salze, mit einer Verbindung der Formel III in 1-Stellung substituiert, wobei Verbindungen der Formel Ie

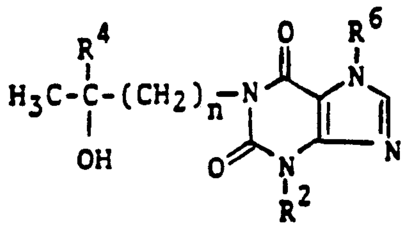

(Ie)

erhalten werden, und jene Verbindungen der Formel Ie, in denen $R^6$ eine Benzyl- oder Diphenylmethylgruppe, oder aber einen Alkoxymethyl- oder Alkoxyalkoxymethyl-Rest darstellt, unter reduzierenden bzw. hydrolytischen Bedingungen in erfindungsgemäße Verbindungen der Formel If

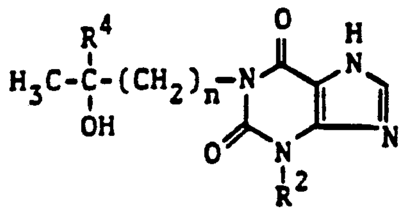

(I f)

überführt, die man gewünschtenfalls nachträglich wieder mit einer Verbindung der Formel III oder IV zu erfindungsgemäßen Verbindungen der Formel Ic bzw. Ie umsetzt, oder

d) erfindungsgemäße Verbindungen der Formel Id bzw. Ie, in denen $R^5$ bzw. $R^6$ einen Oxoalkyl-Rest bedeutet, mit üblichen Reduktionsmitteln an der Ketogruppe zu den entsprechenden erfindungsgemäßen hydroxyalkylierten Xanthinen des Anspruchs 1 reduziert, oder daß man e) substituierte Xanthine der Formel VIII, die

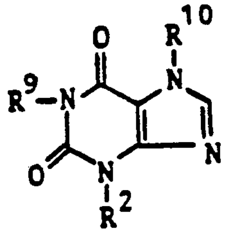

(VIII)

$e_1$) in den Positionen von $R^9$ und $R^{10}$ zwei gleiche oder verschiedene Gruppen der Formel $-(CH_2)_n-CO-CH_3$ (IXa) oder $-(CH_2)_n-CO-R^4$ (IXb) oder aber nur einen Substituenten der Formel IXa oder IXb und in der anderen Stellung Wasserstoff oder den Rest $R^5$ bzw. $R^6$ enthalten, im Falle von IXa mit $(C_1-C_3)$Alkyl— bzw. im Falle von IXb mit Methyl-metall-verbindungen untrer reduktiver "Alkylierung" der Carbonylgruppen zu den erfindungsgemäßen Xanthinen der Formeln Ib bis If umsetzt oder

$e_2$) in den Positionen von $R^9$ und $R^{10}$ zwei gleiche oder verschiedene Gruppen der Formel $-(CH_2)_n-Hal$ (X) oder nur einen derartigen Rest und Wasserstoff oder den Substituenten $R^5$ bzw. $R^6$ in der anderen Position tragen, endständig metalliert und anschließend mit den Ketonen der Formel $R^4-CO-CH_3$ (XI) unter reduktiver Alkylierung der Carbonylgruppe zu den erfindungsgemäßen Xanthinen der Formeln Ib bis If umsetzt oder

$e_3$) in den Positionen von $R^9$ oder $R^{10}$ oder $R^9$ und $R^{10}$ die Gruppe $-(CH_2)_n-COO-(C_1-C_4)$Alkyl (XII) und gegebenfalls Wasserstoff oder den Rest $R^5$ bzw. $R^6$ in der anderen Position tragen, mittels zweier Äquivalente einer Methyl-metall-verbindung pro Alkoxycarbonylfunktion in solche erfindungsgemäßen Xanthine der Formeln Ib bis If überführt, in denen $R^4$ die Bedeutung von Methyl hat, oder

$e_4$) in den Positionen von $R^9$ und $R^{10}$ zwei gleiche oder verschiedene Reste der Formel

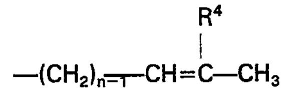

(XIII)

oder nur einen derartigen Rest und in der anderen Stellung Wasserstoff oder den Rest $R^5$ bzw. $R^6$ tragen, wobei die Gruppe XIII die —C=C— Doppelbindung auch in stellungsisomeren Anordnungen am verzweigten C-Atom enthalten kann, durch säurekatalysierte, der Markownikoff-Regel gehorchende Hydratisierung in die erfindungsgemäßen Xanthine der Formeln Ib bis If umwandelt und

$e_5$) gewünschtenfalls anschließend die nach den Methoden $e_1$) bis $e_4$) erhaltenen erfindungsgemäßen tertiären Hydroxyalkylxanthine der Formeln Ib und If, die in 1- bzw. 7-Stellung ein Wasserstoffatom tragen, gegebenenfalls in Gegenwart basischer Mittel oder in Form ihrer Salze, mit den Alkylierungsmitteln der Formel III oder IV bzw. IVa zu den trisubstituierten Verbindungen der Formel Ic bzw. Id oder Ie umsetzt, wobei in den voranstehenden Formeln $R^2$, $R^4$, $R^5$ und n die in Anspruch 1 definierten Bedeutungen haben.

7. Arzneimittel, gekennzeichnet durch einen Gehalt an — oder bestehend aus — mindestens einer Verbindung der Formel I nach einem oder mehreren der Ansprüche 1 bis 5 oder mindestens einer nach dem Verfahren gemäß dem Anspruch 6 hergestellten Verbindung.

8. Arzneimittel nach Anspruch 7, dadurch gekennzeichnet, daß es zur Vorbeugung und/oder Behandlung von peripheren und/oder cerebralen Durchblutungsstörungen verwendet wird.

9. Arzneimittel nach Anspruch 7 oder 8, dadurch gekennzeichnet, daß es zur Vorbeugung und/oder Behandlung der peripheren arteriellen Verschlußkrankheit eingesetzt wird.

10. Arzneimittel nach Anspruch 7, dadurch gekennzeichnet, daß es zur Behandlung von Störungen muskulären Energiestoff-stoffwechsels, insbesondere mitochondrialer Myopathien eingesetzt wird.

**Revendications**

1. Hydroxyalkylxanthines tertiaires de formule générale I

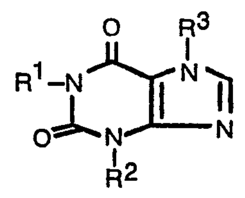

(I)

caractérisées en ce que au moins l'un des radicaux

$R^1$ et $R^3$ représente un groupe hydroxyalkyle tertiaire de formule

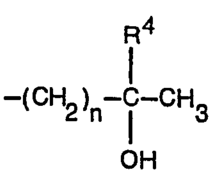

(Ia)

dans laquelle $R^4$ représentent un groupe alkyle ayant jusqu'à 3 atomes de carbone, et n est un nombre entier allant de 2 à 5, et — au cas où un seul des radicaux $R^1$ ou $R^3$, représente un tel groupe hydroxyalkyle tertiaire de formule Ia — l'autre radical représente un atome d'hydrogène ou un radicale hydrocarboné aliphatique $R^5$ ayant jusqu'à 6 atomes de carbone et dont la chaîne carbonée peut être interrompue par

jusqu'à 2 atomes d'oxygène ou peut être substitutée par un groupe oxo ou par jusqu'à deux groupes hydroxy, le groupe oxo ou le(s) groupe(s) hydroxy étant séparé(s) de l'atome d'azote par au moins deux atomes de carbone, et

$R^2$ représente un groupe alkyle ayant de 1 à 4 atomes de carbone.

2. Composés selon la revendication 1, caractérisés par au moins l'une des caractéristiques que $R^2$ représente le groupe méthyle ou éthyle, un seul des deux radicaux $R^1$ ou $R^3$ représente le groupe hydroxyalkyle tertiaire défini dans la revendication 1, et en ce que un groupe oxo ou hydroxy se trouvant sur le radical $R^5$ est séparé de l'atome d'azote par au moins deux atomes de carbone.

3. Composés selon la revendication 1 ou 2, caractérisés en ce que, dans la formule, I, $R^1$ ou $R^3$ représente le groupe [(ω-1)-hydroxy-(ω-1)-méthyl]-pentyle, -hexyle ou -heptyle.

4. Composés selon une ou plusieurs des revendications 1 à 3, caractérisés en ce que, dans la formule I, $R^1$ représente le groupe hydroxyalkyle tertiaire dans lequel, de préférence $R^1$ représente le groupe [(ω-1)-hydroxy-(ω-1)-méthyl]-pentyle, -hexyle ou -heptyle, $R^2$ représente le groupe méthyle ou éthyle et $R^3$ représente un groupe alkyle, hydroxyalkyle ou alcoxyalkyle ayant chacun de 1 à 4 atomes de carbone.

5. Composé selon la revendication 4, caractérisé en ce qu'il représente la 7-éthoxyméthyl-1-(5-hydroxy-5-méthyl-hexyl)-3-méthylxanthine.

6. Procédé pour la préparation de dérivés de xanthine de formule 1 selon une ou plusieurs des revendications 1 à 5, caractérisé en ce que

a) on fait réagir des 3-alkylxanthines de formule II

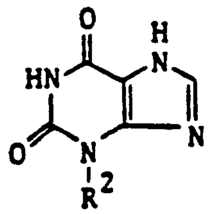

(II)

dans laquelle $R^2$ représente un groupe alkyle ayant jusqu'à 2 atomes de carbone, éventuellement en présence d'agents basiques, ou sous forme de leurs sels, avec des agents d'alkylation de formule III

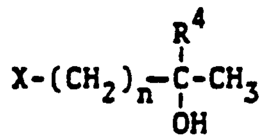

(III)

dans laquelle X est un atome d'halogène ou est un groupement ester sulfonique ou phosphorique, et $R^4$ et n ont les significations données dans la revendication 1, pour aboutir aux composés de formule Ib selon l'invention de formule Ib

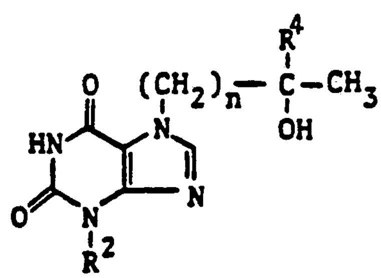

(Ib)

comportant un groupe hydroxyalkyle tertiaire à la position de $R^3$ et un atome d'hydrogène à la position de $R^1$, et, à nouveau de préférence en présence d'agents basiques, ou sous forme de leurs sels,

$a_1$) on soumet ceux-ci à une alkylation avec le même ou avec un autre agent d'alkylation de formule III, pour aboutir aux composés de formule Ic selon l'invention

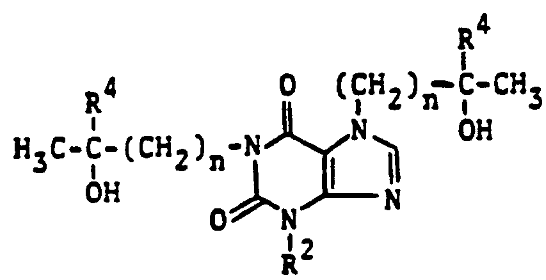

(I c)

comportant deux groupes hydroxyalkyle tertiaires identiques ou différents aux positions de $R^1$ et $R^3$, ou

$a_2$) on les convertit, par réaction avec un composé de formule $R^5$—X (IV), dans laquelle X a la signification donnée à propos de la formule III, et $R^5$ a la signification donnée dans la revendication 1, en composés de formule Id selon l'invention

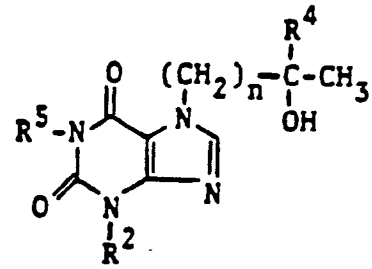

(Id)

ou

b) on soumet à une substitution en position 7 des xanthines 1,3-dialkylées de formule V

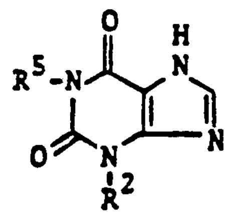

(V)

avantageusement en présence d'agents basiques, ou sous forme de leurs sels, par réaction en une étape avec un composé de formule III, pour aboutir aux composés de formule Id, ou

c) on fait réagir les 3-alkylxanthines de formule II, éaglement de préférence en présence d'agents basiques, ou sous forme de leurs sels, d'abord avec un composé de formule $R^6$—X (IVa) avec formation de xanthines 3,7-disubstituées de formule VI

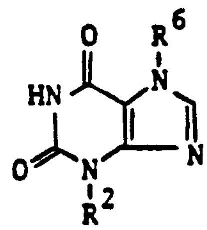

(VI)

dans laquelle $R^6$ a la signification donnée pour $R^5$ ou représente le groupe benzyle ou diphénylméthyle, et X a la signification donnée à propos de la formule III, et on les soumet ensuite à une substitution en position 1, à nouveau de préférence en présence d'agents basiques, ou sous forme de leurs sels, à l'aide d'un composé de formule III, pour obtenir des composés de formule Ie

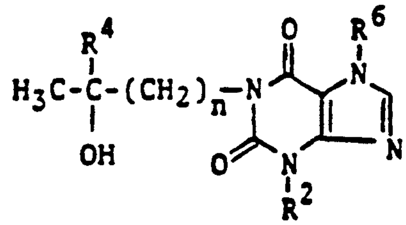

(Ie)

puis on convertit les composés de formule Ie dans lesquels $R^6$ représente le groupe benzyle ou diphénylméthyle ou bien un radical alcoxyméthyle ou alcoxy-alcoxyméthyle, dans des conditions réductrices ou d'hydrolyse, en composés de formule If selon l'invention

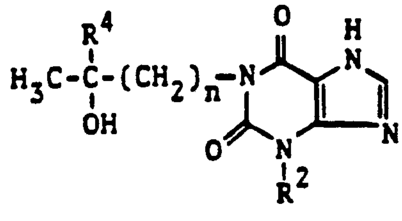

(I f)

que l'on fait ultérieurement réagir, si on le désire, avec un composé de formule III ou IV, pour aboutir aux composés de formule Ic ou, respectivement, Ie, selon l'invention, ou

d) on réduit au niveau du groupe cétonique, à l'aide de réducteurs usuels, des composés de formule Id ou Ie selon l'invention, dans lesquels $R^5$ ou, respectivement, $R^6$, représente un radical oxoalkyle, pour aboutir aux xanthines hydroxyalkylées correspondantes selon l'invention, ou en ce que e) soument des xanthines substituées de formule VIII

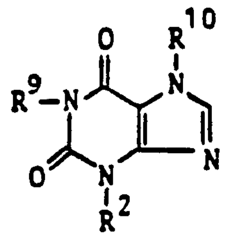

(VIII)

$e_1$) qui comportent aux positions de $R^9$ et $R^{10}$ deux groupes, identiques ou différents, de formule $-(CH_2)_n-CO-CH_3$ (IXa) ou $-(CH_2)_n-CO-R^4$ (IXb), ou bien un seul substituant de formule IXa ou IXb et, à l'autre position, un atome d'hydrogène ou le radical $R^5$ ou $R^6$, à une réaction, dans le cas de IXa, avec des composés alkyl($C_1-C_3$)-métalliques ou, dans le cas de IXb, avec des composés méthylmétalliques, avec "alkylation" réductrice des groupes carbonyle, pour aboutir aux xanthines de formules Ib à If selon l'invention, ou

$e_2$) qui comportent aux positions de $R^9$ et $R^{10}$ deux groupes identiques ou différentes de formule $-(CH_2)_n-Hal$ (X), ou un seul radical de ce type, et un atome d'hydrogène ou le substituant $R^5$ ou $R^6$ à l'autre position, à une métallation en bout de chaîne, et on les fait réagir ensuite avec les cétones de formule $R^4-CO-CH_3$ (XI), avec alkylation réductrice du groupe carbonyle, pour aboutir aux xanthines de formules Ib à If selon l'invention, ou

$e_3$) qui comportent aux positions de $R^9$ ou $R^{10}$ ou de $R^9$ et $R^{10}$ le groupe $-(CH_2)_n-COO-$alkyle($C_1-C_4$) (XII) et éventuellement un atome d'hydrogène ou le radical $R^5$ ou $R^6$ à l'autre position, à une conversion en utilisant deux équivalents d'un composé méthyl-métallique par fonction alcoxycarbonyle, en les xanthines de formules Ib à If selon l'invention, dans lesquelles $R^4$ représente le groupe méthyle, ou

$e_4$) qui comportent aux positions de $R^9$ et $R^{10}$ deux restes, identiques ou différents, de formule

$$-(CH_2)_{n-1}-CH=\overset{R^4}{\overset{|}{C}}-CH_3 \qquad (XIII)$$

ou un seul reste de ce type, et, à l'autre position, un atome d'hydrogène ou le radical $R^5$ ou $R^6$, le groupe XIII pouvant également comporter la double liaison C=C également dans des dispositions d'isomérie de position au niveau de l'atome de carbone ramifié, à une conversion par hydratation catalysée par un acide, obéissant à la règle de Markownikoff, en les xanthines de formules Ib à If selon l'invention, et

$e_5$) éventuellement on fait réagir ensuite les hydroxyalkylxanthines tertiaires de formules Ib et If selon l'invention, obtenues selon les méthodes $e_1$) à $e_4$), qui portent un atome d'hydrogène en position 1 ou 7, éventuellement en présence d'agents basiques, ou sous forme de leurs sels, avec les agents d'alkylation de formule III ou IV ou IVa, pour aboutir aux composés trisubstitués de formule Ic ou Id ou respectivement Ie, $R^2$, $R^4$, $R^5$ et n, dans les formules précédentes, ayant les significations données dans la revendication 1.

7. Médicament caractérisé par une teneur en — ou consistant en — au moins un composé de formule I selon une ou plusieurs des revendications 1 à 5 ou en au moins un composé préparé par le procédé selon la revendication 6.

8. Médicament selon la revendication 7, caractérisé en ce qu'on l'utilise pour la prévention et/ou le traitement de troubles de la circulation périphériques et/ou cérébrale.

9. Médicament selon la revendication 7 ou 8, caractérisé en ce qu'on l'utilise pour la prévention et/ou le traitement de maladies d'occlusion d'artères périphériques.

10. Médicament selon la revendication 7, caractérisé en ce qu'on l'utilise pour le traitement de troubles du métabolisme énergétique musculaire, en particulier de myopathies mitochondriales.

**Claims**

1. A tertiary hydroxyalkylxanthine of the formula I,

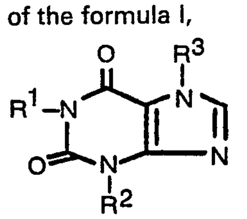

(I)

wherein at least one of the radicals $R^1$ and $R^3$ represents a tertiary hydroxyalkyl group of the formula

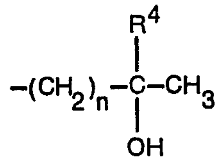

(Ia)

in which $R^4$ denotes an alkyl group with up to 3 carbon atoms, and n denotes an integer from 2 to 5, and, if only one of the radicals $R^1$ or $R^3$ denotes a tertiary hydroxyalkyl group of the formula Ia, the other radical represents a hydrogen atom or an aliphatic hydrocarbon radical $R^5$ with up to 6 carbon atoms, the carbon chain of which can be interrupted by up to 2 oxygen atoms or substituted by an oxo group or up to two hydroxyl groups the oxo group or hydroxy group(s) being separated from the nitrogen by at least 2 carbon atoms, and $R^2$ represents an alkyl group with 1 to 4 carbon atoms.

2. A compound as claimed in claim 1, having at least one of the features that $R^2$ represents methyl or ethyl, only one of the two radicals $R^1$ or $R^3$ represents the tertiary hydroxyalkyl group defined in claim 1, and that a hydroxyl or oxo group present in the radical $R^5$ is separated from the nitrogen by at least two carbon atoms.

3. A compound as claimed in claim 1 or 2, wherein, in the formula I, $R^1$ or $R^3$ denotes [(ω-1)-hydroxy-(ω-1)-methyl]-pentyl, -hexyl or -heptyl.

4. A compound as claimed in one or more of claims 1 to 3, wherein, in the formula I, $R^1$ represents the tertiary hydroxyalkyl group, $R^1$ preferably denoting [(ω-1)-hydroxy-(ω-1)-methyl]-pentyl, -hexyl or -heptyl, $R^2$ preferably denoting methyl or ethyl, and $R^3$ preferably denoting alkyl, hydroxyalkyl or alkoxyalkyl with in each case 1 to 4 carbon atoms.

5. A compound as claimed in claim 4, representing 7-ethoxymethyl-1-(5-hydroxy-5-methylhexyl)-3-methylxanthine.

6. A process for the preparation of xanthine derivatives of the formula I as claimed in one or more of claims 1 to 5, which comprises

a) reacting 3-alkylxanthines of the formula II

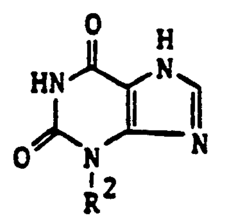

(II)

in which $R^2$ represents alkyl with up to 4 carbon atoms, if appropriate in the presence of basic agents or in the form of their salts, with alkylating agents of the formula III

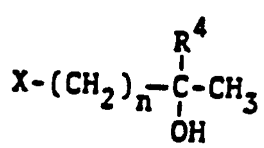

(III)

in which X is halogen or a sulfonic acid ester or phosphoric acid ester grouping and $R^4$ and n have the meanings defined in claim 1, to give compounds of the formula Ib according to the invention

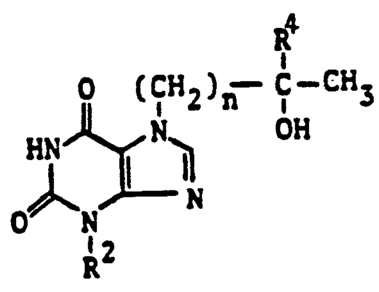

(Ib)

with a tertiary hydroxyalkyl group in the position of $R^3$ and hydrogen in the position of $R^1$, and, again preferably in the presence of basic agents or in the form of their salts,

$a_1$) alkylating these with the same or another alkylating group of the formula III to give compounds of the formula Ic according to the invention

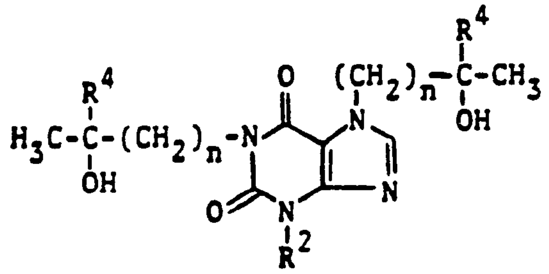

(I c)

with two identical or different tertiary hydroxyalkyl groups in the positions of $R^1$ and $R^3$, or

$a_2$) converting these into compounds of the formula Id according to the invention

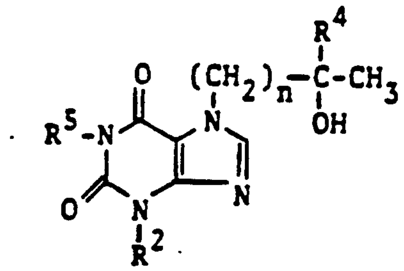

(Id)

by reaction with a compound of the formula $R^5$—X (IV) in which X has the meanings given in the case of the formula III and $R^5$ has the meanings given in claim 1, or

b) substituting 1,3-dialkylated xanthines of the formula V,

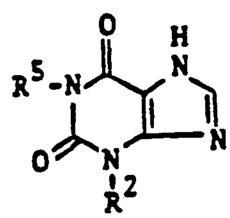

(V)

advantageously in the presence of basic agents or in the form of their salts, in the 7-position to give compounds of the formula Id by a one-step reaction with a compound of the formula III, or

c) first reacting the 3-alkylxanthines of the formula II, likewise preferably in the presence of basic agents or in the form of their salts, with a compound of the formula $R^6$—X (IVa) to form 3,7-disubstituted xanthines of the formula VI

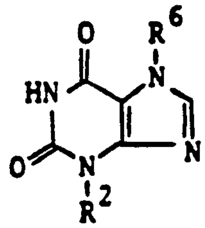

(VI)

in which $R^6$ has the meaning given for $R^5$ or represents benzyl or diphenylmethyl and X has the meaning given in the case of the formula III, and then substituting these, again preferably in the presence of basic agents or in the form of their salts, in the 1-position by a compound of the formula III, compounds of the formula Ie

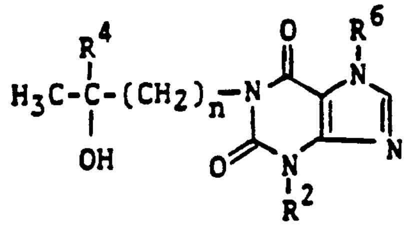

(Ie)

being obtained, and converting those compounds of the formula Ie in which $R^6$ represents a benzyl or

diphenylmethyl group or an alkoxymethyl or alkoxyalkoxymethyl radical into compounds of the formula If according to the invention

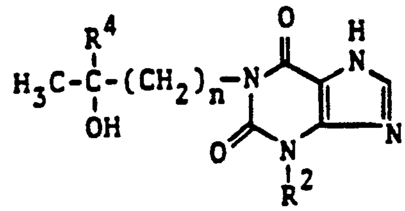

(I f)

under reducing or hydrolytic conditions, and, if desired, subsequently reacting the products again with a compound of the formula III or IV to give compounds of the formula Ic or Ie according to the invention, or

d) reducing compounds of the formula Id or Ie, according to the invention, in which $R^5$ and $R^6$ denotes an oxoalkyl radical, or the keto group with customary reducing agents to give the corresponding hydroxyalkylated xanthines of claim 1 according to the invention, or

e) reacting substituted xanthines of the formula VIII,

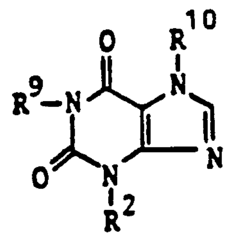

(VIII)

which

$e_1$) contain, in the positions of $R^9$ and $R^{10}$, two identical or different groups of the formula $-(CH_2)_n-CO-CH_3$ (IXa) or $-(CH_2)_n-CO-R^4$ (IXb), or only one substituent of the formula IXa or IXb and, in the other position, hydrogen or the radical $R^5$ and $R^6$, in the case of IXa with ($C_1-C_3$)alkyl- or in the case of IXb with methyl-metal compounds under reductive "alkylation" of the carbonyl groups to form the xanthines of the formulae Ib to If according to the invention, or

$e_2$) metallating, in the terminal position, substituted xanthines of the formula VIII which carry, in the positions of $R^9$ and $R^{10}$, two identical or different groups of the formula $-(CH_2)_n-Hal$ (X) or only one such radical and hydrogen or the substituent $R^5$ or $R^6$ in the other position, and then reacting the products with the ketones of the formula $R^4-CO-CH_3$ (XI), with reductive alkylation of the carbonyl group, to give the xanthines of the formulae Ib to If according to the invention or

$e_3$) converting substituted xanthines of the formula VIII which carry, in the positions of $R^9$ or $R^{10}$ or $R^9$ and $R^{10}$, the group $-(CH_2)_n-COO-(C_1-C_4)$alkyl (XII) and, if appropriate, hydrogen or the radical $R^5$ or $R^6$ in the other position, into those xanthines of the formulae Ib to If according to the invention in which $R^4$ has the meaning of methyl, by means of two equivalents of a methylmetal compound per alkoxycarbonyl group, or

$e_4$) converting substituted xanthines of the formula VIII which carry, in the positions of $R^9$ and $R^{10}$, two identical or different radicals of the formula

$$-(CH_2)_{n-1}-CH=\overset{R^4}{\overset{|}{C}}-CH_3 \qquad (XIII)$$

or only one such radical, and hydrogen or the radical $R^5$ or $R^6$ in the other position, it also being possible for the group XIII to contain the C=C double bond in positionally isomeric locations on the branched carbon atom, into the xanthines of the formulae Ib to If according to the invention by acid-catalyzed hydration obeying the Markovnikov rule, and

$e_5$) if desired, subsequently reacting the tertiary hydroxyalkylxanthines of the formulae Ib and If according to the invention, which carry a hydrogen atom in the 1- or 7-position and are obtained according to methods $e_1$) to $e_4$), if appropriate in the presence of basic agents or in the form of their salts, with the alkylating agents of the formula III or IV or IVa to give the trisubstituted compounds of the formula Ic or Id or Ie, where $R^2$, $R^4$, $R^5$ and n in the above formulae have the meanings defined in claim 1.

7. A medicament which contains or comprises at least one compound of the formula I as claimed in one or more of claims 1 to 5 or at least one compound prepared by the process as claimed in claim 6.

8. A medicament as claimed in claim 7, which is used for the prevention and/or treatment of disturbed peripheral and/or cerebral circulation.

9. A medicament as claimed in claim 7 or 8, which is employed for the prevention and/or treatment of peripheral arterial occlusive disease.

10. A medicament as claimed in claim 7, which is employed for the treatment of muscular energy metabolism disorders, particularly mitochondrial myopathy.